# EUROPEAN PATENT APPLICATION

(11) **EP 4 521 111 A1**
(43) Date of publication of application: **12.03.2025**
(21) Application number: 23195844.8
(22) Date of filing: 06.09.2023
(51) Int. Cl.: G01N 33/53, B01D 15/38, B01J 20/10, C07K 1/14, C07K 1/22, C07K 1/36, G01N 1/34, G01N 33/543, G01N 33/68

(54) **SAMPLE PREPARATION OF BIOLOGICAL SAMPLES FOR ANALYSIS**

(71) Applicant: PreOmics GmbH, 82152 Planegg/Martinsried (DE)
(72) Inventor: KULAK, Nils A., 80686 Munich (DE); Runtsch, Leander S., 82061 Neuried (DE); Pan, Kuan-Ting, 81377 Munich (DE); Cerda, Fabian, 58453 Witten (DE)
(74) Representative: Dentons Patent Solutions Rechtsanwaltsgesellschaft mbH

(57) **Abstract**

The present invention relates to a method of reducing the dynamic range of biomolecules in a biological fluid, said method comprising bringing said biological fluid into contact with fibers; wherein biomolecules as bound to said fibers are characterized in that said dynamic range is reduced as compared to said biological fluid.

## Description

This invention relates to a method of reducing the dynamic range of biomolecules in a biological fluid, said method comprising bringing said biological fluid into contact with fibers; wherein biomolecules as bound to said fibers are characterized in that said dynamic range is reduced as compared to said biological fluid.

In this specification, a number of documents including patent applications and manufacturer's manuals are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

Polypeptide/protein fractionation, separation, or depletion remains a challenge in biopharmaceutical research. For example, one of the major goals in current pharmaceutical research is the identification, detection and quantitation of polypeptide/protein biomarkers out of body fluids such as blood plasma. Because of the very high dynamic range of polypeptides/proteins within bodily fluids, low abundant polypeptides/proteins are often difficult or in many instances impossible to detect with currently available detectors or analyzers. For this purpose, most current methods aim to deplete and remove high abundant polypeptides/proteins such as serum albumin and immunoglobulins by selective binding to proteins which have a higher affinity for the respective protein. For example, Protein-G beads are commonly employed to reduce the quantity of IgG protein in the sample of interest. Furthermore, fractionation methods such as ion-exchange chromatography are also often used to separate polypeptides/proteins and reduce the complexity and dynamic range of the sample.

The concept of selective protein/polypeptide precipitation is well established and was described previously (E. J. Cohn, L. E. Strong, W. L. Hughes, D. J. Mulford, J. N. Ashworth, M. Melin, and H. L. Taylor; Journal of the American Chemical Society 1946 68 (3), 459-475; DOI: 10.1021/ja01207a034). Here the pH, temperature, and ethanol content are utilized to induce a stepwise precipitation of certain polypeptides/proteins in solution in blood plasma. This process remains one of the major processes in large-scale protein enrichment in the pharmaceutical industry to purify certain protein/polypeptides species. This process however requires large amounts of starting material to generate a sufficient precipitate that can be filtered or spun down in order to successfully separate the precipitated polypeptides/proteins from the remaining soluble polypeptides/proteins. Furthermore, automatizing filtration or centrifugation remains challenging.

In view of the shortcomings of the state of the art, the technical problem underlying the present invention can be seen in the provision of improved means and methods for sample processing or sample fractionation, in particular of samples comprising polypeptides or in the field of proteomics including research, manufacturing and diagnostics. Such improved means and methods furthermore shall provide for a reduction of the dynamic range of the analytes to be detected and quantified.

This technical problem has been solved by the enclosed claims and as evidenced by the Examples comprised in this disclosure.

Accordingly, the present invention, in a first aspect, relates to a method of reducing the dynamic range of biomolecules in a biological fluid, said method comprising bringing said biological fluid into contact with fibers; wherein biomolecules as bound to said fibers are characterized in that said dynamic range is reduced as compared to said biological fluid.

In addition or alternatively to reducing said dynamic range, said method is also a method of increasing the number of detectable biomolecules, preferably proteins, polypeptides and/or peptides, in said biological fluid. "Detectable" preferably refers to mass spectrometry. In the context of mass spectrometry, it is preferred to increase the number of unique proteins, polypeptides and/or peptides amenable to detection as compared to non-unique proteins, polypeptides and/or peptides. The term "non-unique" is generally used in the field of MS and refers to the MS data not being indicative of a single ("unique") protein, but rather of a group of (generally related) proteins, where the data are insufficient to resolve said group.

The term "dynamic range" has its art-established meaning in the field of analytics. It refers to the ratio of the amount of most abundant signal in an analyzer to the least abundant signal in a sample, preferably a biological fluid. Large dynamic ratios can compromise the performance of analytical methods, in particular in that the most abundant signal occupy or saturate the detector such that the analytes, which are present in smaller relative amounts, may not be detected or quantifiable. In a biological setting, the interest often focuses on analytes which are present only at lower abundances such as markers for a disease. For that reason, it is desirable to reduce any interfering signal and the dynamic range of analytes present in a sample prior to subjecting them to any analytical method.

The Examples contain evidence of increased detection of low abundance proteins including biomarkers (in particular FDA-approved biomarkers) when using the method of the present invention. In other words, the method of the present invention is also a method of improving the limit of detection (LOD) and/or the limit of quantification (LOQ).

A preferred analytical method is mass spectrometry (MS). Of note, state of the art MS instruments are capable to analyze up to 6 orders of magnitude in dynamic range. The reduction of dynamic range as provided by the present invention is particularly valuable in the field of mass spectrometry.

The term "biomolecules" includes proteins, polypeptides and peptides (as disclosed further below in the context of preferred embodiments), but also embraces nucleic acids such as DNA, RNA and modified versions thereof, lipids, and metabolites, the terms "metabolites" including substances occurring naturally in a given organism as well as molecules which are formed from xenobiotic substances such as medicaments and drugs when being processed by the liver or other organs of such organism. The dynamic range of proteins/polypeptides in blood plasma for instance span more than 10 to 12 orders of magnitude. Comparing these values with those characterizing the capabilities of MS (as given above) it is evident, that reducing the dynamic range of analytes is key in the field of MS.

A "biological fluid" is any fluid originating from an organism. A biological fluid may be a bodily fluid. Preferred embodiments thereof are disclosed further below. A biological fluid may furthermore be a homogenate such as cell or tissue homogenate, a cell lysate, or a solution of biomolecules, in particular of proteins, polypeptides and/or peptides. Said biological fluid may be an unprocessed sample or may be subjected to pre-processing prior to said bringing into contact.

"Fibers" refers to any material made of elongated elements. In many instances, fibers are comprised in or are woven, intertwined, or cross-linked material. As such, fibers in accordance with the invention are generally at least two-dimensional, preferably three-dimensional. In many instances, fibers to used in accordance with the invention are also referred to as "wool" such as glass wool or quartz wool. Of note, wool has to be clearly distinguished in structural terms from particles (as defined further below, and as used in the art). Fibers can be considered as solid-phase extraction material.

"Solid-phase" refers to solid-phase extraction (SPE) technology and materials which have art-established meanings by which compounds that are dissolved or suspended in a liquid mixture are separated from other compounds in the mixture according to their physical and chemical properties. Similarly, the term "matrix" as used herein refers to a three-dimensional composition of matter which is capable of binding biomolecules, in particular proteins, polypeptides and peptides. Such matrix facilitates precipitation of said biomolecules. Preferably, the native state of said biomolecules is preserved upon binding. Preferred implementations of "matrix" include fibers, micro- and nanoparticles, and monoliths or monolithic material.

Fibers preferably do not or do not substantially dissolve in the above-mentioned biological liquids nor in any liquid or reagent to be added to said biological liquids when performing any of the methods of the present invention. Fibers provide a solid-phase binding matrix to which biomolecules can adhere to, preferably non-covalently.

It surprisingly turns out that fibers are capable of differentially binding to biomolecules, wherein low abundance biomolecules are retained in contrast to higher abundant biomolecules, the consequence being that, upon binding to said fibers, the dynamic range of a complex sample such as a biological fluid is reduced. This in turn allows for more analytes being detectable and quantifiable.

In other words, the amount of certain biomolecules is reduced (they would remain, or at least part thereof would remain in a supernatant), while the relative amount of other biomolecules is increased by binding to the fibers, as compared to the original biological fluid. As mentioned above, this improves performance of any subsequent analysis of said biomolecules.

In particular when applied to plasma, it could be shown (see the Examples) that proteins with low abundance in the original sample are enriched upon binding to matrices disclosed herein, in particular, but not only, fibers.

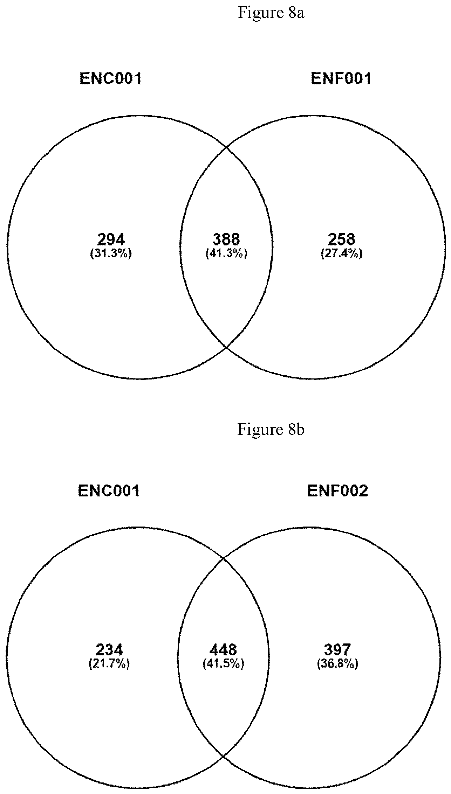

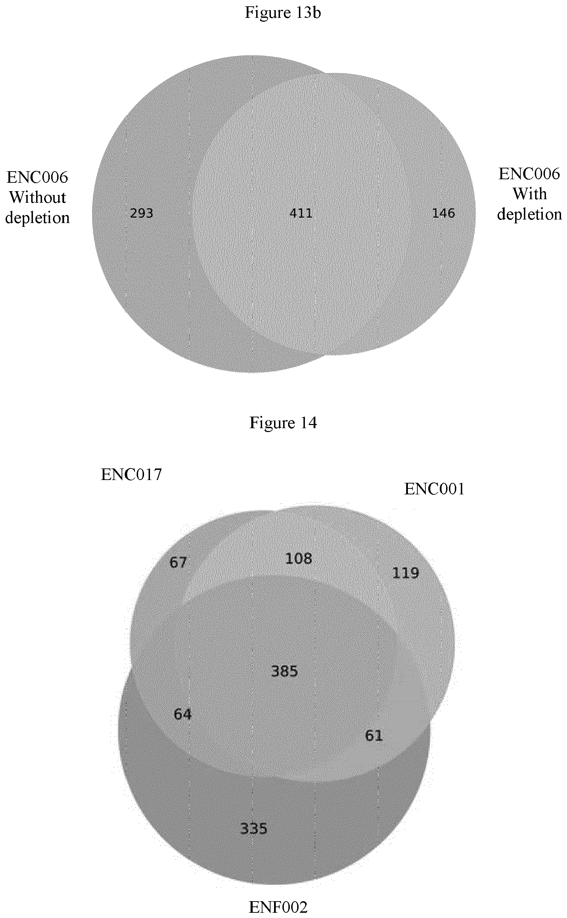

It further turns out that fibers outperform microparticles and nanoparticles which are commonly used for the purpose of fractionating samples such as biological fluids; see, e.g. Example 10 and Figures 8b and 14. Moreover, while the prior art focuses on fractionating samples, the present inventors could demonstrate that the use of fibers not only can be used for fractionating, but also, as explained above, for reducing the dynamic range of a sample.

A further advantage of the use of fibers as solid phase extraction (SPE) material over particles (paramagnetic as well as non-magnetic) is that fibers are easy to handle. Separating fibers with analytes bound does not require any of centrifugation, batch processing, filtration or the use of a magnet. It therefore can be easily automated on a liquid handling platform without the use of specialized equipment such as a centrifuge, positive-pressure unit, vacuum-manifold, or magnetic rack.

Without wishing to be bound by a specific theory, it is suggested that the most abundant biomolecules in a biological fluid tend to be more soluble, while the less abundant biomolecules tend to be less soluble. As such, when a biological fluid is brought into contact with a matrix such as fibers (further and alternative matrices being the subject of further aspects of the present invention as discussed below), it is expected that there is general tendency for the analytes occurring in small relative amounts (low abundance analytes) to adhere to, bind to or associate with said matrix which is higher than the tendency of highly abundant analytes to do so. On such grounds it is expected that the specific chemical or structural nature of said fibers is not decisive when it comes to reducing the dynamic range. The Examples enclosed herewith provide corresponding evidence.

Yet further, the present inventors surprisingly observed that the use of more than one type of fiber (be it different types of base material such as silica, quartz, cotton, metal oxide etc. or be it different types of surface derivatizations, to the extent such derivatizations are present which is not a requirement) provide for a yet further reduction or differentiation in the specificity of the dynamic range and/or an increase of the number of analytes being amenable to detection (generally also referred to as "depth", especially but not only in the field of proteomics). Such uses of more than one type of fiber may be concomitantly, i.e., in one pot, parallel, or may be subsequently, "subsequently" preferably being in the sense that results of a first performance of the method of the invention (e.g., "result" being the supernatant or eluate of analytes bound to said matrix such as fibers; "methods" referring to any of the methods disclosed herein) are subjected to a further performance (or a plurality of further performances) of the methods of the invention, wherein for one or more of said subsequent performances a different type of matrix or fibers is used.

The mentioned adherence, binding or association of biomolecules as comprised in biological fluids with a matrix such as fibers (but also including particles and monoliths as disclosed further below) can occur under a variety of conditions from which a skilled person can choose.

It is understood that said bringing under contact is done under suitable conditions. Such conditions are binding conditions. They allow for binding of at least part of the biomolecules to the fibers. In other words, it is understood that said method involves allowing binding of said biomolecules to said fibers.

In the simplest and preferred case, a buffered aqueous solution is used (also referred to as "binding buffer" herein). Such solution is provided in a vessel where the methods of the present invention are performed. Preferably said solution has a pH value between 7 and 11, more preferably between 7 and 10, yet more preferably between 8 and 10. Any suitable buffering substance may be used. Examples include Tris, TAPS, Bicine, Tricine, TAPSO, HEPES, TES, MOPS, PIPES, cacodylate, MES, phosphate, DIPSO, MOBS, HEPPSO, POPSO, TEA, EPPS, Gly-Gly, Bicin, HEPBS, AMPD, TABS, AMPSO, CHES, CAPSO, AMP, CAPS, CABS, Bis-Tris Propan, boric acid, Taurine, and carbonate buffers with a pH value ranging from 8 to 11.

A preferred temperature for said bringing into contact is between about 20 and about 40°C, more preferably about 30°C.

The term "about" as used herein generally means +/- 20%, +/-10%, or +/-5%. In any case, it accommodates the measurements errors commonly observed for the parameter in question.

Preferred time ranges for bringing into contact are between about 1 to about 120 min, about 2 to about 60 min, about 5 to about 30 min, and more preferred about 30 min.

These preferred parameters for said bringing into contact apply to all aspects of the invention.

In addition, conditions controlling binding behaviors to said matrix and/or precipitation may be used. Among the conditions reducing binding and/or precipitation of high abundant analytes, preference is given to mild conditions as spelled out in the following. In particular, said conditions may involve a change of the physicochemical conditions of the sample to be processed, in particular of said biological fluid. Further details in that respect can be found in the section entitled "Physicochemical conditions for binding" further below.

In a further preferred embodiment, said biological liquid has been treated with an anticoagulant.

Treatment of biological samples with anticoagulants is common in the art, in particular when it comes to clinical samples. To give an example, while serum is the liquid component of blood which may coagulate when stored, the addition of anticoagulants serves to prevent coagulation and gives rise to plasma. The blood clot in case of serum and the blood cells in case of plasma are generally removed by centrifugation. Anticoagulants are known in the art, wherein EDTA is particularly common, EDTA K2 and EDTA K3 being preferred. Related to said first aspect, the present invention also provides a method of processing, fractionating and/or preparing for analysis biomolecules in a biological fluid, said method comprising bringing said biological fluid into contact with fibers. The above given definitions and explanations as well as preferred implementations given below apply mutatis mutandis.

Also related to said first aspect, the present invention provides a method of reducing the amount of interfering molecules, reducing the amount of interfering and high abundant molecules, and/or reducing the relative quantity of high abundant molecules in a biological fluid, said method comprising bringing said biological fluid into contact with fibers. The above given definitions and explanations as well as preferred implementations given below apply mutatis mutandis.

The notion of interfering molecules is discussed further below in relation to a preferred embodiment. Generally, the term relates to molecules which deteriorate the performance of a downstream analytical method (here preferably MS).

Said interfering molecules are enriched or retained in the supernatant.

Also related to the first aspect, the present invention relates to a method of processing, fractionating or preparing for analysis a sample comprising biomolecules such as proteins, polypeptides and/or peptides, said method comprising (a) changing the physicochemical conditions of said sample; and (b) bringing fibers into contact with said sample; wherein steps (a) and (b) can be effected concomitantly or in any order; and wherein said method yields one or more first fractions of biomolecules as a precipitate on said fibers, and a second fraction of biomolecules remaining in a supernatant. Preferably, said sample is a biological fluid. The above given definitions and explanations as well as preferred implementations given below apply mutatis mutandis.

The further purposes and technical effects as recited in the above aspects apply to the other aspects of the present invention as well, especially those directed to methods and uses, wherein said methods and uses, as disclosed further below, may use other matrices such as particles or monoliths or any combination thereof.

The term "fractionating" has its art-established meaning. It refers to dividing a mixture of compounds or analytes into at least two separate mixtures, wherein said separate mixtures differ in their relative contents with regard to at least one of said compounds or analytes. In other words, at least one analyte is enriched in at least one fraction as compared to at least one other fraction. Fractionating may lead to at least one analyte being substantially or totally depleted from at least one fraction but does not have to. In a more general sense, fractionation may lead to a modified or decreased dynamic range or complexity as compared to the original mixture, e.g., the ratio of the amount of most abundant analyte to the amount of the least abundant analyte may be decreased in at least one of the obtained fractions as compared to the original mixture. Such decrease in dynamic range will generally increase the amount of analytes amenable to detection in subsequent analytical procedures, e.g. mass spectrometry (MS). The term "complexity" as used here refers to the number of analytes, in particular biomolecules, more specifically proteins, polypeptides and or peptides comprised in a given fraction. A compression of the dynamic range generally entails a higher number of analytes being detected.

Preferably, and as stated above, said biomolecules are proteins, polypeptides and/or peptides.

The terms "protein", "polypeptide" and "peptide" have their art-established meanings as well. In particular, peptides and polypeptides are polycondensates of amino acids, preferably of the proteinogenic L-alpha-amino acids. Herein, such polycondensate is referred to as peptide if it contains 30 or less amino acids, and longer polycondensates are referred to as polypeptides. Preferred peptides are those with a length from 20 to 30 amino acids. Generally, peptides and polypeptides are single chains, which does not exclude the presence of cross-links such as disulfides and/or modifications such as phosphorylation. Proteins on the other hand may have a more complex structure in that they may be oligomers of polypeptides and/or comprise non-proteinaceous components such as prosthetic groups. The analysis of such proteins, polypeptides and peptides is of particular interest when it comes to distinguishing healthy from diseased states at a molecular level.

Preferably, said biological fluid is selected from bodily fluid, cell lysate or processed forms thereof, said processed forms thereof including the result of chromatography such as size exclusion chromatography (SEC), ion exchange chromatography, affinity chromatography or any combination thereof.

These are optional means for the depletion of high abundance proteins prior to performing the method of the invention. They may be used under certain circumstances, but generally, and as shown in the Examples, there no such requirement. Affinity chromatography for depletion of high abundant proteins, e.g., in plasma, is well established and includes Protein A affinity chromatography.

In some embodiments, said method comprises analysis and/or quantitation of said biomolecules as bound to said fibers, wherein preferably said analysis and/or quantitation is done by mass spectrometry.

Preferably, said fibers are selected from mineral fibers such as silica fibers, preferred silica fibers being glass fibers and quartz fibers; organic or textile fibers such as cellulose, cotton, wool, hemp, linen, proteins and polymers; and metal or metal oxide fibers.

Preferred are silica fibers and organic or textile fibers. More specifically, preferred are silica-based fibers with or without additional chemical functionalization, wherein the functionalization is selected from reversed-phase material, normal-phase material, cation-exchange (strong and weak) material, anion-exchange (strong and weak) material, chelating material, and/or combinations thereof such as mixed-phase materials, preferably ion exchange/normal phase mixed-phase materials.

Particularly preferred fibers are silica fibers.

The term "silica" generally refers to pure or substantially pure SiO2. In addition, silica has on its surface free Si-OH groups. Therefore, silica itself qualifies as a normal phase material in accordance with the invention. Furthermore, said SiOH groups are functional groups which lend themselves for chemical derivatization. Such derivatized materials based on silica are the subject of preferred embodiments as discussed herein. They include the Acidosil material disclosed further below.

As opposed to silica, quartz may contain further minerals in addition to SiO2. Therefore, while quartz may be viewed as a silica-type material in a wider sense, it is chemically distinct to a certain degree. Having said that, sometimes "quartz" is subsumed under "silica" in a wider sense.

Example 1 contains a table presenting further preferred fibers which have been used in the examples.

In some embodiment, said biomolecules are (a) eluted from said fibers after said bringing into contact; or (b) cleaved while being bound to said fibers.

In order to subject biomolecules as bound to a matrix such as fibers to analysis, e.g., by MS, unbinding of the analytes to be subjected to analysis is required.

Such unbinding or eluting may be performed by adding an eluent to the matrix-analyte complex. Exemplary eluents and, more generally, conditions favoring unbinding are disclosed further below in the section entitled "Eluting".

Having said that, in a further preferred embodiment, such dissociation or unbinding is not necessary and/or not performed. In particular, said biomolecules, especially protein, polypeptides or peptides, as bound to said fibers (or any other matrix such as particles or monoliths disclosed herein) may be subjected to cleavage in its bound state.

Such on-matrix cleavage is advantageous in that it provides for a further simplification of the workflow of sample (biological fluid) preparation for subsequent analytics and/or quantification.

Cleavage may be with one or more protease, or, less preferred, with a chemical.

In a further particularly preferred embodiment, (i) said protease is selected from serine proteases, serine carboxy proteases, cysteine proteases, aspartic proteases, metalloproteases and metallocarboxy proteases, preferably from trypsin, chymotrypsin, Lys-C, Lys-N, Asp-N, Glu-C and IdeS; (ii) said chemical is HCl, BrCN, BNPS-skatole, formic acid, hydroxylamine, or 2-nitro-5-thiocyano benzoic acid; or (iii) mechanical cleaving is by means of at least one moving magnet present in said supernatant or eluate, respectively.

Having regard to proteases, the recited classes are EC classes as follows: serine proteases (EC 3.4.21), serine carboxy proteases (EC 3.4.16), cysteine proteases (EC 3.4.22), aspartic proteases (EC 3.4.23), metalloproteases (EC 3.4.24), and metallocarboxy proteases (EC 3.4.17).

As mentioned above, the methods of the invention, in particular said bringing into contact will generally be done in a vessel.

Preferred vessels are microtiter plates. Examples include 384 well plates with 45 ul or 140 ul volume per well, and 96x well plates with volumes of 150 ul, 200ul, 250 ul, 350ul, 500ul, 1000ul, or 2000ul per well.

Also suitable are tubes such Eppendorf or falcon tubes, or strips (preferred volumes being 500 ul, 1000 ul, 1500ul, 2000 ul, 5000ul, 15000ul, 50000ul).

Any other type of vessel is envisaged. Any vessel volume in an interval defined by any pairs of values given above (such as from 45 ul to 50000ul) as well as larger or smaller volumes are considered.

In some embodiments, (a) more than one type of fiber is used; or (b) in addition to at least one type of fiber, microparticles, nanoparticles and/or monoliths are used.

Preferably, said microparticles or nanoparticles are mixed-phase particles and/or a porous with a pore width of about 10 to 100 nm, more preferably 10 to 50 nm, still more preferably 10 to 20 nm.

Such particles are discussed in more detail further below in relation to further aspects of the present invention.

For example, more than one type of fiber may be provided in a single vessel. This is also referred to as "one-pot workflow" in the present disclosure. It surprisingly turned out the use more than one type of fiber in a single performance of the method further improves outcome, in particular in terms of dynamic range and/or the number of analytes being amenable to detection. An example of such workflow is described in Example 1.

In another workflow, the same or different types of fibers are provided in different vessels. After performing the method of the invention in a first vessel, the supernatant is transferred to a second vessel. Such "sequential workflow" can involve also more than two performances of the method (and accordingly employ more than two vessels. Preference is given to the use of a different type of fiber in each vessel. Effectively, such workflow amounts to fractionation: with each performance of the method, in the sample (biological fluid in the beginning, thereafter supernatant from the previous performance of the method) analytes are reduced in number and/or amount, thereby reducing complexity of the sample, reducing dynamic range and/or increasing the number of analytes being detectable. Preferably, and as stated below, data obtained from an analytical method such as MS from each matrix-analyte complex are combined at the data level. This provides an enhanced dataset which is more comprehensive as compared to a single performance of the method of the invention. An example of such workflow is described in Example 1.

Yet another preferred workflow, which also subject of a separate aspect of the invention (see further below), is referred to as "sequential crossed workflow". In that case, two different matrices (such as fibers) are provided in separate vessels. Aliquots of the same sample (bodily fluid) are added to each vessel, respectively. After performing the method of the invention, the supernatants are removed and subsequently transferred to the respective other vessel. An example of such workflow is described in Example 1. This novel type of workflow lead to the following surprising finding: The results of the subsequent use of different matrices such as fibers are dependent on the order in which said matrices are used. Combining the results at the data level, which preferably is being done, provides an enhanced dataset which is more comprehensive as compared the (plain) sequential use of different matrices, i.e., without "crossing", crossing meaning the reciprocal transfer of supernatants to the respective other vessel. Instead of two aliquots of the sample, two different samples may be used.

A further workflow is the following "parallel workflow": Different matrices such as fibers are provided in different vessels. After performing the method of the invention in each vessel with an aliquot of a sample (bodily fluid), the results (matrix-analyte complexes and/or supernatants) are subjected to analysis, preferably by MS. Preferably, data obtained from analysis are combined at the data level.

A workflow which is particularly suitable for reducing the number of non-unique proteins is the following "depletion workflow": Use is made of at least three different matrices (fibers, particles, monoliths or combinations thereof). At least two or all possible combination of two matrices out of said three matrices are provided in separate vessels. After a first performance the method of the invention in each of the three vessels, each of the respective supernatants is transferred to a separate new vessel, wherein in each new vessel that particular matrix out of the three matrices is provided which was absent from the first performance of the method. Subsequently, the method is performed a second time with the respective supernatants in the respective new vessels. This workflow is also subject of a separate aspect of the invention disclosed further below.

Whenever reference is made to aliquots of the same sample, two different samples may be used instead.

Also, different samples may be combined prior to processing them with the method of the invention.

Different samples may be the same biological fluid processed differently or differently labelled, e.g., isotope labelled. Different samples may originate from different organisms or the same organism at different points in time. Different samples may differ with regard to health state (such as healthy vs. diseased) or with regard to treatment status (such as untreated vs. treated, e.g., with a medicament or drug). Different samples may be different types of biological fluid.

Instead of supernatants being transferred in the context of the above embodiments and workflows, fibers may be transferred into another vessel instead or in addition. Any suitable may be used for that purpose, e.g., tweezers.

In some embodiments, said method is repeated with the same or a different type of fiber, wherein, instead of said biological fluid, an eluate from the fibers or a supernatant as obtained in a preceding performance of said method is used; and/or different aliquots of biological fluid are used for each repetition of said method.

As discussed further below, analytical data obtained from such several performances of said method may be combined.

In some embodiments, (i) a supernatant is removed, preferably by pipetting, aspirating or decanting only and/or removing said supernatant does not involve centrifugation; and/or (ii) addition or removal of liquids such as reagents is done by pipetting, aspirating or decanting only and/or does not involve centrifugation.

In some embodiments, said method comprises analyzing the data obtained from said analysis and/or quantitation.

In some embodiments, said analyzing comprises combining and/or comparing the said data obtained from a plurality of performances of the method the method of the first aspect.

As demonstrated in the Examples, such combining at the data level allows for increased depth, i.e., the number analytes being detected is increased as compared to the absence of such combining.

Preferably, said bodily fluid is selected from plasma, serum, full blood, cerebrospinal fluid, saliva, nasal swab, urine, synovial fluid, vaginal fluid and semen.

In some embodiments, fibers are derivatized with functional groups.

In some embodiments, said fibers comprise an outer layer comprising or consisting of material selected from mixed-phase material, said mixed phase material preferably being combined normal phase material; ion exchange material; reversed-phase material; normal phase material; a chelator; and any combination thereof.

Preferably, said fibers have normal phase and ion exchange properties, normal phase properties preferably being provided by the fibers being made of silica, quartz or glass, and ion exchange being preferably selected from WCX, SCX, WAX and SAX.

Further exemplary functional groups include:
R-CH3, R-CH2-CH3, R-(CH2)3-CH3, R-(CH2)4-CH3, R-C6H5, R-C5H4N, R-COOH, R-COOOH, R-CS-OH, R-SO2-OH, R-SO-OH, R-S-OH, R-COO- M+, R-SO2-O- M+, R-SO-O-M+, R-SO- M+, RO-NO2, R-CO-NH2, R-SO2-NH2, R-CO-NH-NH2, R-C=N, R-CO-H, R-CS-H, R1R2-C=N-OH, R1R2-C=N-NH2, R-OH, R-SH, R-NH2, R-NH-NH2, R-N+-R1R2R3, wherein R1, R2 and R3 independently represent a hydrogen atom, an alkyl, an aryl or a vinyl and M represents a counter ion, and R is a group or free valence on the fiber to be derivatized.

Embodiments and exemplary implementation of the method of the first aspect apply mutatis mutandis to the following further aspects of the invention.

In some embodiments, furthermore the amount of substances interfering with analysis is reduced upon binding, said interfering substances including salts, sugars, amino acids, and, to the extent said biomolecules are proteins, polypeptides and/or peptides, said interfering substances include furthermore nucleic acids and lipids.

Such interfering substances would be enriched in the supernatant as compared to the matrix-bound, more specifically fibers-bound state.

In a second aspect, the present invention provides the use of fibers for reducing the dynamic range of biomolecules in a biological fluid.

Preferred fibers are those disclosed in context of the discussion of the method of the first aspect above. The same applies to "biomolecules" and "biological fluid".

In a third aspect, the present invention provides a method of reducing the dynamic range of proteins, polypeptides and/or in a biological fluid, said method comprising:
bringing said biological fluid into contact with microparticles or nanoparticles in a buffered aqueous solution with a pH between 7 and 10;
wherein the particles are mixed-phase particles; and
wherein proteins as bound to the particles are characterized in that they are in their native state and that said dynamic range is reduced.

Buffering compounds useful for preparing said buffered aqueous solution are disclosed herein above. Preferred pH values include a pH value between 7 and 11, more preferably between 7 and 10, yet more preferably between 8 and 10.

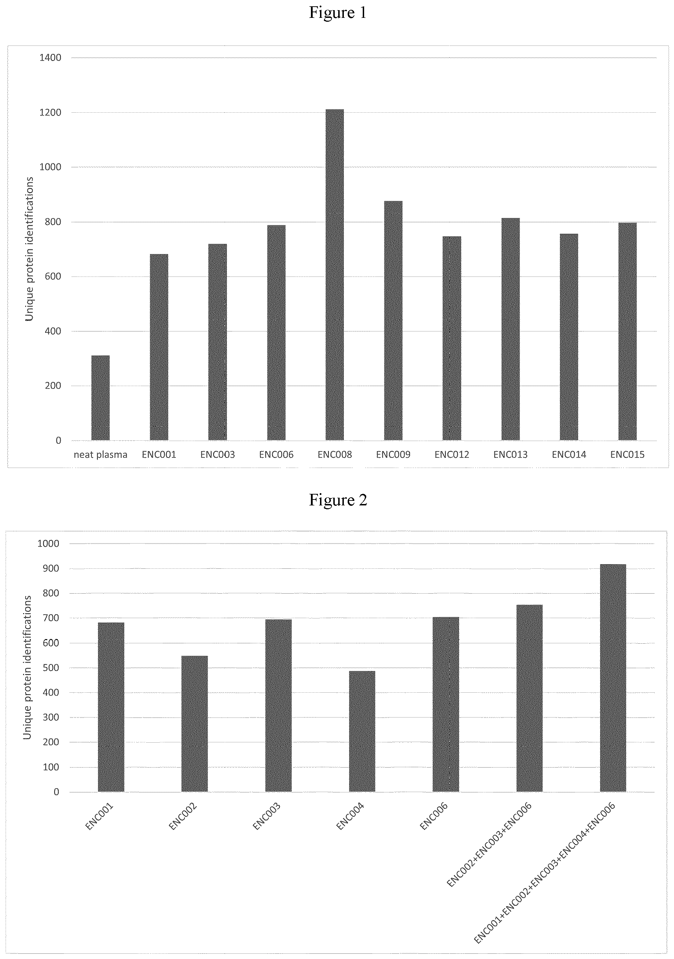

The inventors found that mixed-phase particles outperform normal phase particles. Corresponding evidence can be found in, for example, in Example 2 and Figure 1.

Micro- and nanoaparticles, especially for analytical and sample preparation purposes are well established in the art. This is solid particulate matter equipped with a surface capable of interacting with analytes. Micro- and/or Nanoparticles as disclosed herein, are commercially available in a wide variety of materials, including ceramics, glass, polymers, and metals. The general conception of the shape of a particle (microparticle and nanoparticles) is a spherical or sphere-like, which is in contrast with the general definition of fibers above. Exemplary particle sizes are given in Example 1.

In a preferred embodiment, said microparticles or nanoparticles are paramagnetic. This can be implemented by a paramagnetic core. Paramagnetic particles can be handled (e.g., separated from a supernatant) by using a magnet.

Preferably, said mixed-phase particles have normal phase and ion exchange properties, normal phase preferably being silica and ion exchange being preferably selected from WCX, SCX, WAX and SAX. This applies to all matrices considered herein, including fibers and monoliths.

WCX stands for weak cation exchange and may be implemented with a weak acidity, preferably carboxylates bound to the solid phase (preferably silica), optionally with a linker.

SCX stands for strong cation exchange and may be implemented with a strong acidity, preferably sulfonates bound to the solid phase (preferably silica), optionally with a linker.

WAX stand for weak anion exchange and may be implemented with a weak base, preferably a primary-, secondary-, or teriary-amine group bound to the solid phase (preferably silica), optionally with a linker.

SAX stand for weak anion exchange and may be implemented with a strong base, preferably a quaternary-ammonium groups bound to the solid phase (preferably silica), optionally with a linker.

Generally, anion exchange resins consist of a polymeric matrix to which different functional groups are attached. Most weakly basic anion exchangers contain tertiary amino groups; in a few cases primary and secondary groups are also encountered. In many cases weakly basic anion exchangers are not monofunctional but possess a variety of amino groups. Strongly basic resins contain quaternary ammonium groups. Standard commercially available exchangers contain either -N+(CH3)3 groups (type 1 resins) or -N+(CH3)2C2H4OH groups (type 2 resins). Both weakly and strongly basic exchange resins are available in gel-type or macro-porous modifications.

Normal phase materials are functionalized with hydroxy-, diol-, cyano- and aminofunctional groups or exposed silica surface of a silica solid phase.

A preferred embodiment is silica surface. Example 1 presents a table with preferred silica particles which have been used in the Examples.

Particularly preferred are silica particles derivatized with either Si-C3H6-NH-CO-CH2-CH2-COOH or Si-C3H6-O-CH2-COH2-CH2-SO3H groups on their surface. Such particles are also known as Acidosil-C and Acidosil-S particles, respectively. Generally, a significant number of free Si-OH groups on the silica surface is not derivatized in these particles.

Also considered are silica particles which are derivatized as follows: Si-C3H6-O-CH2-COH2-CH2-NR¹R², wherein R¹ and R² are independently H or C₁ to C₄ alkyl; or Si-C3H6-O-CH2-COH2-CH2-NR¹R²R³ wherein R³ is defined as R¹ and R² and the nitrogen carries positive charge.

Further exemplary functional groups include:
R-CH3, R-CH2-CH3, R-(CH2)3-CH3, R-(CH2)4-CH3, R-C6H5, R-C5H4N, R-COOH, R-COOOH, R-CS-OH, R-SO2-OH, R-SO-OH, R-S-OH, R-COO- M+, R-SO2-O- M+, R-SO-O-M+, R-SO- M+, RO-NO2, R-CO-NH2, R-SO2-NH2, R-CO-NH-NH2, R-C=N, R-CO-H, R-CS-H, R1R2-C=N-OH, R1R2-C=N-NH2, R-OH, R-SH, R-NH2, R-NH-NH2, R-N+-R1R2R3, wherein R1, R2 and R3 independently represent a hydrogen atom, an alkyl, an aryl or a vinyl and M represents a counter ion, and R is a group or free valence on the particle to be derivatized.

The above disclosed mixed-phase derivatizations of normal phase material (such as silica) can also be present on the fibers as disclosed further above.

More preferably, the particles are porous, preferably with a pore width of about 5 to 100 nm, preferably 10 to 50 nm, more preferably of about 10 to 20 nm, even more preferably of about 12 to 15 nm. Given that manufacturers generally specify pore widths in Angstrom, it is noted that 1 nm = 10 Angstrom.

In a fourth aspect, the present invention provides a method of reducing the dynamic range of proteins, polypeptides and/or peptides in a biological fluid, said method comprising: bringing said biological fluid into contact with microparticles or nanoparticles in a buffered solution with a pH value between about 7 and about 10;
wherein the particles are porous with a pore size of about 10 to 20 nm, preferably about 12 to 15 nm; and
wherein proteins, polypeptides and/or peptides as bound to the particles are characterized in that they are in their native state and that said dynamic range is reduced.

Preferred pH values include a pH value between 7 and 11, more preferably between 7 and 10, yet more preferably between 8 and 10.

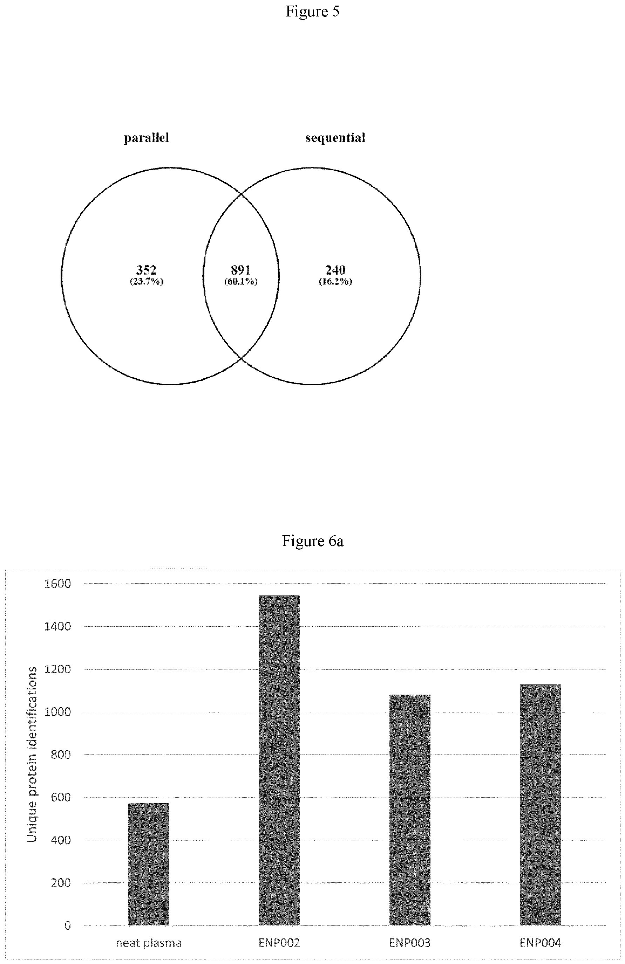

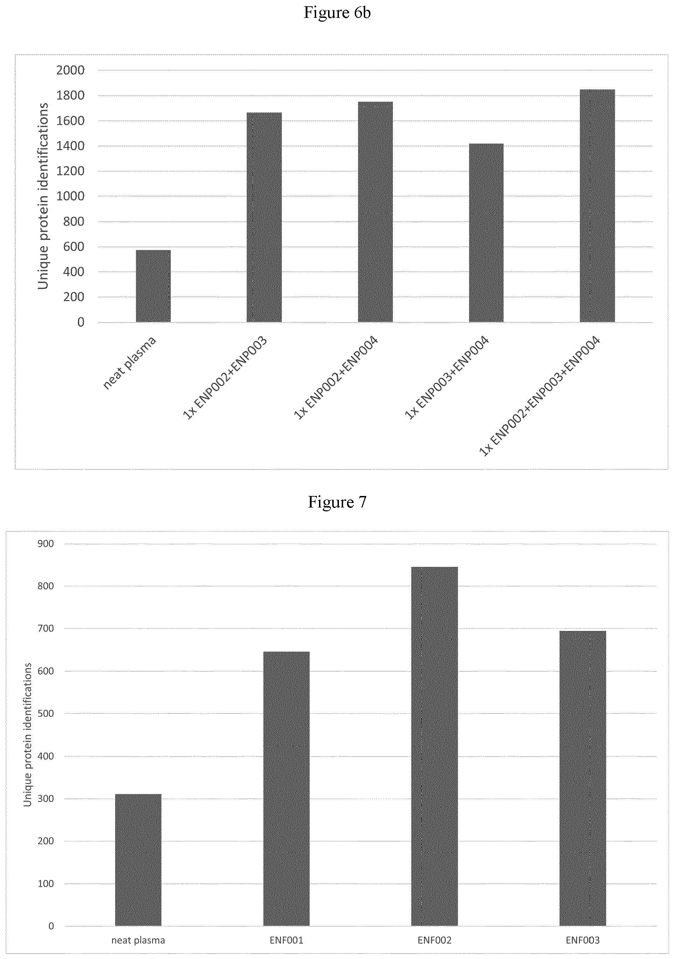

This aspect relates to pore sizes of demonstrated performance; see Example 7 and Figures 6a and 6b.

In some embodiments, different types of particles and/or particles with different pore sizes are used.

In a fifth aspect, the present invention provides a method of reducing the dynamic range of proteins, polypeptides and/or peptides in a biological fluid, said method comprising: bringing said biological fluid into contact with monoliths in a buffered solution a pH value between about 7 and about 10;
wherein biomolecules as bound to said monoliths are characterized in that said dynamic range is reduced.

Monoliths consist of organic polymer- or inorganic oxide-based monolithic media, which form porous rod structures characterized by mesopores and macropores. The macropores provide high permeability, allowing significantly faster flow rates. The mesopores form a fine porous structure, which creates a very large active surface area for high-efficiency separations.

Examples of polymeric monoliths are monolithic media obtained from synthetic (polymethacrylate, polyacrylamide, and polystyrene) and natural (agarose and cellulose) polymers. Polymeric monoliths generally withstand pH extremes and are compatible with most non-chlorinated solvents. However, despite a high degree of cross-linking, many of these polymer substrates still suffer some shrinkage and swelling in the presence of certain organic solvents. They are rarely stable at temperatures above ca. 200 °C. Polymeric monolithic media can be chemically functionalized via co- or post-polymerization of functional monomers or imprinting techniques.

On the other hand, inorganic oxide-based monolithic media, such as metal oxides of Zr, Si, Ti, Al, Hf, C, Au, Ag, Ce, or Ge, can be stable at extremely high temperatures (over 750 °C) but cannot be operated in pH extremes.

As such, preferred monoliths comprise or consist or oxide of an element selected from Si, Zr, Ti, Al, Hf, C, Au, Ag, Ce, and Ge; or of a polymer selected from polymethacrylate, polyacrylamide, polystyrene, agarose and cellulose.

Monoliths can be chemically modified for specific applications, for example monoliths can be functionalized to include chemical groups, such as R-CH3, R-CH2-CH3, R-(CH2)3-CH3, R-(CH2)4-CH3, R-C6H5, R-C5H4N, R-COOH, R-COOOH, R-CS-OH, R-SO2-OH, R-SO-OH, R-S-OH, R-COO- M+, R-SO2-O- M+, R-SO-O- M+, R-SO- M+, RO-NO2, R-CO-NH2, R-SO2-NH2, R-CO-NH-NH2, R-C=N, R-CO-H, R-CS-H, R1R2-C=N-OH, R1R2-C=N-NH2, R-OH, R-SH, R-NH2, R-NH-NH2, R-N+-R1R2R3 wherein R1, R2 and R3 independently represent a hydrogen atom, an alkyl, an aryl or a vinyl and M represents a counter ion, and R is a group or free valence on the monolith to be derivatized.

Preferably, said monoliths have normal phase and ion exchange properties, normal phase preferably being silica, for example provided by the monolith itself comprising or consisting of silica, and ion exchange being preferably selected from WCX, SCX, WAX and SAX.

In the present invention monoliths have been found to be surprisingly useful for the separation of high and low abundance proteins contained in a biological fluid. In accordance with the present invention, provided is their use in any means of biological sample separation materials, the separation material exemplified by, but not limited to spin columns, cartridges, multi-well plates, and pipette tips.

Most preferably, said monoliths comprise or consist of silica.

Such monoliths or monolithic material can be obtained, for example, from Kyoto Monotech Co., Ltd.

Preferred pH values include a pH value between 7 and 11, more preferably between 7 and 10, yet more preferably between 8 and 10.

In a sixth aspect, the present invention provides a method of reducing the dynamic range of proteins, polypeptides and/or peptides in a sample and/or increasing the number of detectable proteins, polypeptides and/or peptides in a sample, said method comprising:
(a) adding an aliquot of said sample to each of two vessels, wherein each vessel contains a different binding matrix;
(b) allowing binding of proteins, polypeptides and/or peptides to each matrix;
(c) removing the supernatants from each vessel; and
(d) adding the supernatant removed from the first vessel of said two vessels to the second vessel and vice versa.

This aspect defines the sequential crossed workflow as already discussed further above.

In a seventh aspect, the present invention provides A method of reducing the dynamic range of proteins, polypeptides and/or peptides in a sample and/or increasing the number of uniquely detectable proteins, polypeptides and/or peptides in a sample, said method comprising:
(a) providing three different matrices in six different vessels as follows:
   vessel 1: matrices 1 and 2
   vessel 2: matrix 3
   vessel 3: matrices 2 and 3
   vessel 4: matrix 1
(b) adding an aliquot of said sample to each of vessels 1 and 3;
(c) allowing binding of proteins, polypeptides and/or peptides to the matrices in each of the vessels of (b); and
(d) transferring the supernatants from vessels 1 and 3 to vessels 2 and 4, respectively.

This aspect defines a depletion workflow related to the one discussed further above.

Preferred embodiments of the methods of the sixth and seventh aspect are as follows:

Preferably, said matrices are selected from fibers, micro- or nanoparticles and monoliths.

Preferably, at least one matrix is selected from fibers and monoliths, and/or the particles are silica particles with either Si-C3H6-NH-CO-CH2-CH2-COOH or Si-C3H6-O-CH2-COH2-CH2-SO3H groups on their surface.

Preferably, the methods further comprise:
(e) allowing binding of proteins, polypeptides and/or peptides as comprised in the supernatants of (d) to the matrices in each of the vessels, where said supernatants have been added or transferred to.

More preferably, the methods further comprise:
(f1) eluting said proteins, polypeptides and/or peptides from said matrices and proteolytically digesting them; or
(f2) proteolytically digesting them while being bound to said matrices; and optionally
(g) analyzing the results of (f1) or (f2) by mass spectroscopy.

In an eighth aspect, the present invention provides a kit comprising or consisting of: (a) a protease; (b) a reducing agent; (c) an alkylating agent; and (d) a silica particle with an outer layer comprising or consisting of material selected from mixed-phase material, said mixed phase material preferably being combined normal phase material; ion exchange material; reversed-phase material; normal phase material; a chelator; and any combination thereof.

In a ninth aspect, the present invention provides a kit comprising or consisting of: (a) a protease; (b) a reducing agent; (c) an alkylating agent; and (d) silica fibers, preferably glass wool or quartz wool.

In a tenth aspect, the present invention provides a kit comprising or consisting of: (a) a protease; (b) a reducing agent; (c) an alkylating agent; and (d) a monolith comprising or consisting of silica.

In some embodiments of the ninth and tenth aspect, said fibers or said monolith, respectively, comprise an outer layer comprising or consisting of material selected from mixed-phase material, said mixed phase material preferably being combined normal phase material; ion exchange material; reversed-phase material; normal phase material; a chelator; and any combination thereof.

Said normal phase or normal phase component of combined or mixed-phase material may be provided by said silica.

Ion exchange materials include anion exchange (weak or strong) and cation exchange (weak or strong) material as discussed in more detail further above.

In addition, said kits may comprise a binding buffer. It is understood that each of the above disclosed kits contains constituents which are useful for performing methods and uses of the invention. As such, it is understood that said binding buffer is preferably the aqueous buffered solution, preferably with a pH value between about 7 and about 10 as discussed in relation to the methods. Further conditions suitable for binding are disclosed in the below section "physicochemical conditions for binding".

Protease, reducing agent and alkylating agent are reagents typically needed for sample preparation for bottom-up mass spectrometry. In other words, any of the above kits is "for sample preparation for mass spectrometry", "sample" including biological fluids, in particular bodily fluids as disclosed above.

Reducing agents and alkylating agents, in particular those for sample preparation for mass spectrometry, are known to the skilled person. Preferred reducing agents include TCEP. Preferred alkylating agents are iodoacetamide and chloroacetamide. Particularly preferred is chloroacetamide (CAA).

In a preferred embodiment, said fibers are selected from the group of fibers consisting of Glass fiber, Quartz fiber, Cotton fiber, Viscose fiber, Sheep fiber, Silk fiber, Nylon fiber, Polyester fiber, Acrylic fiber, Polyethylene fiber, Polypropylene fiber, Aramid fiber, Polytetrafluoroethylene fiber, Ceramic fiber, Aluminium fiber, and Carbon fiber; preferably Glass fiber, Quartz fiber, and Cotton fiber; more preferably Glass fiber, and Quartz fiber; even more preferably Glass fiber.

In a particularly preferred embodiment, said fibers are glass fibers or quartz fibers, and said fibers are functionalized with a functional group selected from R-CH3, R-CH2-CH3, R-(CH2)3-CH3, R-(CH2)4-CH3, R-C6H5, R-C5H4N, R-COOH, R-COOOH, R-CS-OH, R-SO2-OH, R-SO-OH, R-S-OH, R-COO- M+, R-SO2-O- M+, R-SO-O- M+, R-SO- M+, RO-NO2, R-CO-NH2, R-SO2-NH2, R-CO-NH-NH2, R-C=N, R-CO-H, R-CS-H, R1R2-C=N-OH, R1R2-C=N-NH2, R-OH, R-SH, R-NH2, R-NH-NH2, R-N+-R1R2R3 wherein R1, R2 and R3 independently represent a hydrogen atom, an alkyl, an aryl or a vinyl and M represents a counter ion, and R is a group or free valence on the fibers to be derivatized.

In a preferred embodiment, said monolith comprise or consist or oxide of an element selected from Si, Zr, Ti, Al, Hf, C, Au, Ag, Ce, and Ge; or of a polymer selected from polymethacrylate, polyacrylamide, polystyrene, agarose and cellulose, and preferably comprise or consist of silica (SiO2).

Optionally, said silica is functionalized with a functional group selected from R-CH3, R-CH2-CH3, R-(CH2)3-CH3, R-(CH2)4-CH3, R-C6H5, R-C5H4N, R-COOH, R-COOOH, R-CS-OH, R-SO2-OH, R-SO-OH, R-S-OH, R-COO- M+, R-SO2-O- M+, R-SO-O- M+, R-SO- M+, RO-NO2, R-CO-NH2, R-SO2-NH2, R-CO-NH-NH2, R-C=N, R-CO-H, R-CS-H, R1R2-C=N-OH, R1R2-C=N-NH2, R-OH, R-SH, R-NH2, R-NH-NH2, R-N+-R1R2R3 wherein R1, R2 and R3 independently represent a hydrogen atom, an alkyl, an aryl or a vinyl and M represents a counter ion, and R is a group or free valence on the monolith to be derivatized.

In an eleventh aspect, provided is a kit comprising or consisting of at least two different fibers, wherein the first and second fiber are independently selected from the group consisting of Glass fiber, Quartz fiber, Cotton fiber, Viscose fiber, Sheep fiber, Silk fiber, Nylon fiber, Polyester fiber, Acrylic fiber, Polyethylene fiber, Polypropylene fiber, Aramid fiber, Polytetrafluoroethylene fiber, Ceramic fiber, Aluminium fiber, and Carbon fiber; preferably Glass fiber, Quartz fiber, and Cotton fiber; more preferably Glass fiber, and Quartz fiber. Glass and quartz fibers may be derivatized as disclosed above.

In a twelfth aspect, provided is a kit comprising or consisting of at least two different silica particles,
wherein said different particles are selected from particles having an outer layer comprising or consisting of material selected from mixed-phase material, said mixed phase material preferably being combined normal phase material; ion exchange material; reversed-phase material; normal phase material; a chelator; and any combination thereof;
   and/or
wherein said particles are selected from particles carrying a functional group, the functional group being selected from the group consisting of Diol, CN, NH2, 4-Ethylpyridin, Propyl-strong anion exchange, Si-C3H6-NH-CO-CH2-CH2-COOH, Si-C3H6-O-CH2-COH2-CH2-SO3H, Propyl-strong cation exchange, Carboxymethyl, Methyl, Ethyl, Butyl, Butyl+polar groups, Pentyl, and Phenyl; preferably Si-C3H6-NH-CO-CH2-CH2-COOH, and Si-C3H6-O-CH2-COH2-CH2-SO3H; more preferably Si-C3H6-O-CH2-COH2-CH2-SO3H, R-CH3, R-CH2-CH3, R-(CH2)3-CH3, R-(CH2)4-CH3, R-C6H5, R-C5H4N, R-COOH, R-COOOH, R-CS-OH, R-SO2-OH, R-SO-OH, R-S-OH, R-COO- M+, R-SO2-O- M+, R-SO-O- M+, R-SO- M+, RO-NO2, R-CO-NH2, R-SO2-NH2, R-CO-NH-NH2, R-C=N, R-CO-H, R-CS-H, R1R2-C=N-OH, R1R2-C=N-NH2, R-OH, R-SH, R-NH2, R-NH-NH2, R-N+-R1R2R3,
wherein R1, R2 and R3 independently represent a hydrogen atom, an alkyl, an aryl or a vinyl and M represents a counter ion, and R is a group or free valence on the particle to be derivatized.

In a thirteenth aspect, provided is a kit comprising or consisting of at least two different monoliths, wherein each of said monoliths is selected from monoliths comprising or consisting of an oxide of an element selected from Si, Zr, Ti, Al, Hf, C, Au, Ag, Ce, and Ge; or of a polymer selected from polymethacrylate, polyacrylamide, polystyrene, agarose and cellulose, and preferably comprise or consist of silica.

Optionally at least one of said monoliths has a modified surface layer or is derivatized as disclosed further above in conjunction with monoliths.

Yet further aspects of the invention relates to the following kits:
A kit comprising or consisting of at least one type of fibers and one type of monolith.

A kit comprising or consisting of at least one type of fibers and one type of particles.

A kit comprising or consisting of at least one type of monolith and one type of particles.

A kit comprising or consisting of at least one type of fibers, at least one type of particles, and at least one type of monoliths.

Preferred embodiments of fibers, particles, and monoliths as given in this disclosure apply to these aspects.

### Physicochemical conditions for binding

"Physicochemical conditions" embrace both physical parameters such as temperature as well as chemical parameters such as solvent composition. Physicochemical conditions have an influence on the state of analytes in a mixture which state may be expressed in terms of the chemical potential of an analyte.

Said physicochemical conditions to be changed in accordance with the invention are one, more or all of: (i) pH value; (ii) temperature; (iii) concentration of at least one organic solvent; (iv) concentration of at least one salt; (v) concentration of at least one surfactant; and (vi) concentration of said protein(s), polypeptide(s) and/or peptide(s).

In a particularly preferred embodiment, the change of pH in step (a) is towards the isoelectric point of the protein(s), polypeptide(s) and/or peptide(s) to be precipitated on said matrix, in particular fibers. It is known that proteinaceous molecules are least soluble in the vicinity of their isoelectric point (IEP). It is also preferred to establish a pH from 8 to 11, such as from 8 to 10 or from 8 to 9.

Agents which change pH are not particularly limited and include acids and bases.

Preferred acids include inorganic acids such a boric acid, sulfuric acid, nitric acid and hydrogen halides including HF, HCl and HBr; sulfonic acids such as methane sulfonic acid and benzene sulfonic acid; carboxylic acids such as formic acid, acetic acid and citric acid; halogenated carboxylic acids such as trifluoro acetic acid, chloroacetic acid and fluoroacetic acid.

Preferred bases include metal oxides, metal hydroxides, metal carbonates and metal bicarbonates. Particularly preferred bases are NaOH, KOH, LiOH, CsOH, Mg(OH)₂, Ca(OH)₂, Sr(OH)₂, Ba(OH)₂ and furthermore NH₃, tetramethlyammonium hydroxide, guanidine, butyl lithium and NaH.

In a particularly preferred embodiment, the change in temperature in step (a) is a decrease. A decrease of temperature generally decreases solubility which affects different proteinaceous analytes to different extents.

The term "organic solvent" has its art-established meaning. It embraces both protic and aprotic polar organic solvents.

In a particularly preferred embodiment, the change of concentration of an organic solvent is an increase or decrease, preferably of an alcohol such as ethanol, methanol, n-propanol, i-propanol; a nitrile such as acetonitrile; a ketone such as acetone; a glycol ether such as 2-methoxy ethanol; an aliphatic hydrocarbon such as hexane; an aromatic hydrocarbon such as benzene; an aldehyde such as formaldehyde; or mixture thereof.

If the sample to be processed is free or essentially free of organic solvents, an increase of concentration in step (a) is obviously a preferred option.

On the other hand, if the sample to undergo precipitation contains an organic solvent, for example because it has undergone pre-processing involving the addition of an organic solvent, decreasing the concentration of said organic solvent may also be a means of fine-tuning and/or optimizing precipitation conditions.

Yet, and since the method of the invention can be applied to unprocessed biological fluids, it will generally an increase in the concentration of said organic solvent.

In a particularly preferred embodiment, the change in concentration of a salt in step (a) is an increase in concentration of said salt, said salt preferably being a kosmotropic salt.

"Salting out" using kosmotropic salts is known as a means to trigger precipitation. Preferred kosmotropic salts include ammonium sulfate, potassium phosphate, and salts with a cation selected from , Li+, Zn2+ and Al3+ and an anion selected from carbonate, sulfate and hydrogen phosphate.

In a further particularly preferred embodiment, the change in concentration of a salt in step (a) is a decrease in concentration of said salt, said salt preferably being a chaotropic salt. Chaotropic salts generally increase solubility. Therefore, a decrease in concentration is preferred. Preferred chaotropic salts are NaCl, MgCl₂, guanidinium salts such as guanidinium hydrochloride, perchlorates such as lithium perchlorate, lithium acetate, Barium salts, thiocyanates, iodides and sodium dodecyl sulfate (SDS).

The different effect of different salts/cations/anions on precipitation is known in the art as the Hofmeister series.

In a particularly preferred embodiment, the change in concentration of a surfactant is an increase in concentration. Adding a surfactant is a means to keep protein, polypeptides and peptides in solution which otherwise have an undesirably high tendency to precipitate. This applies, for example, to transmembrane proteins such as apolipoproteins.

To the extent said sample comprises surfactants or has undergone pre-processing by adding surfactants, it is also envisaged to decrease the concentration of a surfactant in step (a). This would generally be a means to trigger precipitation.

Preferred surfactants are non-denaturing surfactants, in particular those disclosed further below; see the section entitled "surfactants".

In a particularly preferred embodiment, the change of concentration of said protein(s), polypeptide(s) and/or peptide(s) in step (a) is an increase. Such increase may be achieved, e.g. by evaporation.

In an alternative particularly preferred embodiment, the change of concentration of said protein(s), polypeptide(s) and/or peptide(s) in step (a) is a decrease, e.g. by adding solvent such as water or buffer (e.g. PBS). Preferably, said solvent to be added mimics the composition of the sample (disregarding said proteins, polypeptides and peptides).

In a further preferred embodiment, said change of physicochemical conditions is selected from: (i) an increase in pH, preferably to yield a pH value from 7 to 12 such as from 7 to 11 or from 7 to 10, (ii) a rise in temperature, wherein said temperature after said raise is lower than 50°C preferably up to 40°C; (iii) an increase in concentration of an organic solvent, wherein said concentration of said solvent after said increase is less than 20% (v/v); and (iv) an increase in concentration of a chaotropic salt, wherein said concentration of said chaotropic salt after said increase is less than 0.5 M.

As regards pH, in some embodiment a pH value from 8 to 10 is preferred.

Furthermore, an increase in concentration of a surfactant, albeit less preferred, is envisaged.

The above preferred embodiment provides specific definitions of what is more generally referred to as "less harsh" or "non-denaturing" conditions herein.

Particularly preferred temperatures after said raise are less than 40°C, less than 30°C or no raise at all in which case it is preferred to keep said sample at ambient temperature (such as 20°C) or below.

Particularly preferred concentrations of said organic solvent are below 15% (v/v), below 10% (v/v), or below 5% (v/v) which includes absence of organic solvents.

Preferred chaotropic salts are disclosed further above. Particularly preferred concentrations of said chaotropic salts are below 0.4 M, below 0.3 M, below 0.2 M, below 0.1 M, below 0.05 M or below 0.01 M, including the absence of chaotropic salts.

Within surfactants or detergents, a distinction can be made as regards their effect on structural and functional integrity of protein, polypeptides and peptides. Denaturing surfactant include sodium dodecyl sulfate (SDS). A further denaturing surfactant is ethyl trimethyl ammonium bromide.

Owing to their less harsh properties, non-denaturing surfactants are employed, preferably with concentrations below 5%, below 4%, below 3%, below 2% or below 1%, all (w/v).

Furthermore, a compound selected from PEG, glycerol and DMSO may be added. These compounds act as stabilizing agents.

### Eluting

The above mentioned eluate may be obtained by establishing conditions which favor a dissociation or unbinding from said fibers.

In a preferred embodiment, such conditions are established by adding a detergent, a solvent such as trifluoro ethanol (TFE), a chaotropic agent such as urea or thiourea, an acid such as trifluoro acetic acid (TFA), a base such as NaOH, or a mixture thereof.

In some embodiments, such conditions are established by (i) an increase in the concentration of a surfactant, preferably of a non-denaturing surfactant; (ii) a change in concentration of an organic solvent, wherein the total concentration of organic solvents after said change is less than 20% (v/v); (iii) a change in pH, preferably to yield a pH from 6 to 12, more preferably from 8 to 11, and yet more preferably from 9 to 11 or 10 to 11; (iv) a change in temperature; and/or (v) the addition of a compound selected from PEG, glycerol and DMSO.

The "increase in the concentration of a surfactant" is also referred to herein as "eluting" or the addition of an "elution solution" to the analytes in their bound state. Preferred concentrations of surfactants, in particular non-denaturing surfactants, in said elution solution are from 0.1 to 10 % (w/v), preferably 0.5 to 5 % (w/v) such as 1, 1.5 or 2 % (w/v).

Preferred surfactant concentrations in the solution to be added to said precipitate may also be expressed in terms of the critical micelle concentration (CMC). The CMC is the concentration of a given surfactant above which said surfactant forms micelles. CMC values are generally specified by the manufacturers of surfactants. Preferably, CMCs are reported for aqueous solutions (without salts or other additives) at 25°C.

In a preferred embodiment, the concentration of a given surfactant in said solution is between 0.5 times and 200 times the CMC of said surfactant, more preferably between 1 times and 50 times the CMC or between 1 times and 10 times the CMC such as between 1.5 times and 4 times the CMC or between 1.5 times and 2 times the CMC.

**Table 1 below shows the CMCs for certain preferred surfactants.**

| Detergent | MW | CMC |
|---|---|---|
| CHAPS | 614.88 | 8 mM |
| CHAPSO | 630.88 | 8 mM |
| BIGCHAP | 878.06 | 2.9 mM |
| DeoxyBIGCHAP | 862.06 | 1.4 mM |
| Octylglucoside | 292.37 | 25 mM |
| Heptylthioglucoside | 294.41 | 30 mM |
| Octylthioglucoside | 308.44 | 9 mm |
| Decylmaltoside | 482.57 | 1.8 mM |
| Dodecylmaltoside | 510.62 | 0.17 mM |
| Nonylthiomaltoside | 484.60 | 2.4 mM |
| MEGA-8 | 321.41 | - |
| MEGA-9 | 335.44 | 25 mM |
| MEGA-10 | 349.46 | 7 mM |
| Sucrose monocholate | 732.85 | 4.7 mM |
| Sodium cholate | 448.57 | 14 mM |

Both denaturing surfactants and non-denaturing surfactants may be used.

When all or substantially of the bound proteins, polypeptides and peptides are to be eluted, preference will be given to denaturing surfactants such as SDS.

If only a fraction of the bound proteins, polypeptides and peptides are to be eluted, preference will be given to non-denaturing surfactants.

In a particularly preferred embodiment, (i) said non-denaturing surfactant is selected from deoxycholates; cholates; sulfobetaines; C₄ to C₁₆ alkyl or alkenyl glycosides or thioglycosides, sugar in said glycosides or thioglycosides preferably being glucose or maltose; NP-40; Triton X-100; Triton X-114; Brij-35; Brij-68; Tween-20; Tween-80; Digitonin; and mixtures thereof; or (ii) in case said surfactant is a denaturing surfactant, the concentration of said denaturing surfactant after said increase is below 0.5% (w/v).

Preferred sub-classes of the classes of compounds mentioned in item (i) are defined further above as are particularly preferred specific compounds.

The term "concentration of said denaturing surfactant after said increase" in accordance with item (ii) of this embodiment refers to the concentration in the sample or fraction after adding said denaturing surfactant.

As stated above, cholates and deoxycholates such as SC and SDC are particularly preferred non-denaturing surfactants.

PEG, glycerol and DMSO provide solubilizing and/or stabilizing activity. Generally, the addition of protein-stabilizing agents such as those mentioned here is preferred.

In a further preferred embodiment, reducing agents are not used or, if used, in concentrations below 0.1% (w/v). Reducing agents include Tris(2-carboxyethyl)phosphine (TCEP) and dithiothreitol (DTT).

### Surfactants

Preferred are non-denaturing surfactants, in particular:
cholates and deoxycholates such as sodium deoxycholic acid (also referred to as sodium deoxycholate, SDC) and sodium cholate (SC);
sulfobetaines such as 3-[(3-Cholamidopropyl)dimethylammonio]-1-propanesulfonate (CHAPS), 3-([3-Cholamidopropyl]dimethylammonio)-2-hydroxy-1-propanesulfonate (CHAPSO), 3-(4-(1,1-bis(hexyloxy)ethyl)pyridinium-1-yl)propane-1-sulfonate (PPS), sodium 3-((1-(furan-2-yl)undecyloxy)carbonylamino)propane-1-sulfonate (ProteaseMAX), sodium 3-[(2-methyl-2-undecyl-1,3-dioxolan-4-yl)methoxy]-1-propanesulfonate (RapiGest SF), 3-(Decyldimethylammonio) propanesulfonate, inner salt (DPS);
C4 to C16 alkyl or alkenyl glycosides or thioglycosides, sugar in said glycosides or thioglycosides preferably being glucose or maltose, in particular:
   C4 to C14 alkyl or alkenyl glucosides including C6 to C12 n-alkyl or n-alkenyl glucosides such as n-hexyl β-D-glucoside, n-heptyl β-D-glucoside, n-octyl β-D-glucoside (OGP), n-nonyl β-D-glucoside, n-decyl β-D-glucoside, and n-dodecyl β-D-glucoside;
   C4 to C14 alkyl or alkenyl thioglucosides including C6 to C12 n-alkyl or n-alkenyl thioglucosides such as n-heptyl β-D-thioglucoside, and n-octyl β-D-thioglucopyranoside (OTG);
   C6 to C16 alkyl or alkyenyl maltosides including C8 to C14 n-alkyl or n-alkenyl maltosides such as n-octyl β-D-maltoside, n-nonyl β-D-maltoside, n-decyl-β-D-maltopyranoside (DM), n-decyl α-D-maltoside, n-undecyl β-D-maltoside, n-undecylenyl β-D-maltoside, n-dodecyl-β-D-maltopyranoside (DDM), and n-tridecyl β-D-maltoside;
   C6 to C16 alkyl or alkyenyl thiomaltosides including C8 to C14 n-alkyl or n-alkenyl thiomaltosides such as n-octyl β-D-thiomaltoside, n-nonyl β-D-thiomaltoside, n-decyl β-D-thiomaltoside, and n-undecyl β-D-thiomaltoside;
2-[4-(2,4,4-trimethylpentan-2-yl)phenoxy]ethanol (Triton X-100), Triton X-114;
Brij-35, Brij-68;
Tween-20, Tween-80;
polyethylene glycol nonylphenyl ether (NP-40);
Digitonin;
and mixtures thereof.

Triton X-100 is also known as polyethylene glycol p-(1,1,3,3-tetramethylbutyl)-phenyl ether. It generally has 9 to 10 ethylene oxide units on average.

Triton X-114 is also known as (1,1,3,3-tetramethylbutyl)phenyl-polyethylenglycol. It generally has 7 to 8 ethylene oxide units on average.

Brij-35 is also known as polyoxyethylene lauryl ether. It generally has about 23 ethylene oxide units on average.

Brij-68 is also known as polyoxyethylene cetyl ether. It generally has about 20 ethylene oxide units on average.

Tween-20 and Tween-80 are also known as polyoxyethylene (20) sorbitan monolaurate and Polyoxyethylene (80) sorbitan monooleate, respectively, or polysorbate 20 and polysorbate 80, respectively.

NP-40 is also known as polyethylene glycol nonylphenyl ether. It generally has 9 to 10 ethylene oxide units on average.

Non-denaturing sulfobetaines (NDSBs) are described, for example, in Vuillard et al., Biochem J 305, 337-343 (1995).

Further surfactants which may be employed include:
Octyl maltoside, fluorinated
ANAPOE^{®} NID-P40
1,2-Diheptanoyl-sn-Glycero-3-Phosphocholine
Big CHAP, deoxy
C12E8
ANAPOE^{®}-C10E9
CYCLOFOS^{®}-4
CYGLU-3
CYMAL^{®}-6
n-Dodecyl-β-D-maltoside
Dimethyldodecylphosphine oxide
FOS-Choline^{®}-10
HECAMEG
n-Hexyl-β-D-glucoside
MEGA-10
n-Decyl-a-D-maltoside
DDMAB
n-Nonyl-β-D-glucoside
n-Octyl-β-D-glucoside
CHAPS
n-Undecyl-N,N-Dimethylamine-Oxide
TWEEN^{®} 80
n-Decanoylsucrose
ZWITTERGENT^{®} 3-08
1,2-dioctanoyl-sn-glycero-3-phosphocholine
2,6-Dimethyl-4-heptyl-β-D-maltoside
C6E3
C8E5
ANAPOE^{®}-C13E8
CYCLOFOS^{®}-6
CYMAL^{®}-3
Decyl Maltose Neopentyl Glycol
Lauryl Maltose Neopentyl Glycol
FOS-CholineS-12
Genapol^{®} X-100
n-Heptyl-β-D-glucopyranoside
Dimethylnonylphosphine oxide
n-Decyl-N,N-Dimethylamine-Oxide
n-Decyl-β-D-thiomaltoside
n-Heptyl-β-D-thioglucoside
n-Octyl-β-D-maltoside
Nonidet P40
FOS-Choline^{®}-14
C-HEGA-11
ZWITTERGENT 3-16
TRIPAO
ANAPOE^{®}-20
Omega-Undecylenyl-β-D-Maltoside
LysoFos Choline 10
MEGA-9
ANAPOE^{®}-35
C10E5
CHAPSO
CYMAL^{®}-4
CY-TRIPGLU
n-Decyl-β-D-maltoside
Dimethylundecylphosphine oxide
FOS-Choline^{®}-ISO-11
HEGA-10
Façade-EM
LAPAO
n-Decyl-N,N-dimethylglycine
n-Dodecyl-N,N-dimethylglycine
n-Tridecyl-β-D-maltoside
n-Undecyl-β-D-maltoside
Ph-TRIPGLU
Sodium deoxycholate
ZWITTERGENT^{®} 3-10
C8E4
C8E6
ANAPOE^{®}-X-114
ANAPOE^{®}-X-305
C6E4
C7E5
Octyl Glucose Neopentyl Glycol
C12E9
ANAPOE^{®}-C12E10
FOS-Choline^{®}-UNSAT-11-10
CYMAL^{®}-7
MEGA-8
n-decyl-β-D-glucoside
n-Dodecyl-N,N-dimethylamine-N-oxide
n-Dodecyl-a-D-maltoside
n-Nonyl-β-D-maltoside
n-Octyl-β-D-thiomaltoside
n-Undecyl-β-D-thiomaltoside
OHES
1,2-Dihexanoyl-sn-Glycero-3-Phosphocholine
LysoFos Choline 12
RDHPOS
Sodium cholate hydrate
Sodium dodecyl sulfate
ZWITTERGENT^{®} 3-12
n-Dodecyl β-D-glucoside
ANAPOE^{®} X-100
PCC-a-maltoside
BIGCHAP; and
Sucrose monocholate

Preferred are mild surfactants, i.e., surfactants which do not denaturate proteins, polypeptides and peptides, but solubilize them or portions thereof which are hydrophobic and would aggregate in absence of surfactants. All non-denaturing surfactants among the lists of surfactants given above are preferred. Such surfactants include cholates and deoxycholates such as SDC and SC as well as those given further below.

The Figures show:
- Figure 1.: Unique proteins identified by use of microparticles functionalized with different chemical groups.
- Figure 2.: Unique proteins identified by combining functionalized microparticles with different chemical groups in one-pot.
- Figure 3.: Unique proteins identified by combining the proteomic data obtained from different microparticles incubated sequentially with a sample.
- Figure 4.: Unique proteins identified by combining the proteomic data obtained from different microparticles (in parallel).
- Figure 5.: Venn diagram that shows unique protein identifications obtained by 2 different workflows, sequential and in-parallel. In both cases the method was performed combining the proteomic data derived from the use of the same three types of microparticles (ENC002, ENC003, and ENC006).
- Figure 6a.: Unique proteins identified by use of microparticles with different pore sizes.
- Figure. 6b.: Unique proteins identified by use of a combination of proteomics data obtained from microparticles with different pore sizes.
- Figure 7.: Unique proteins identified by use of different types of fibers.
- Figure 8a.: Venn diagram shows unique protein identifications obtained by use of silica microparticle ENC001 and silica-based fiber ENF001.
- Figure 8b.: Venn diagram shows unique protein identifications obtained by use of silica microparticle ENC001 and silica-based fiber ENF002.
- Figure 9.: Unique proteins identified by combining the proteomic data obtained from different types of fibers.
- Figure 10a.: Abundance of proteins identified in neat plasma. Grey circles (aggregated in a line) represent protein ID's found in the Human Proteome Atlas for which its abundance in human plasma is known. Dark circles represent the protein ID's identified with in neat plasma without the use of fibers or microparticles by the method disclosed herein.
- Figure 10b.: Abundance of proteins identified in plasma processed using fiber ENF002. Grey circles (aggregated in a line) represent protein ID's found in the Human Proteome Atlas for which its abundance in human plasma is known. Dark circles highlight the protein ID's identified using the fiber ENF002.
- Figure 10c.: Abundance of proteins identified by combining the proteomic data obtained from processing plasma with different types of fibers. Grey circles (aggregated in a line) represent protein ID's found in the Human Proteome Atlas for which its abundance in human plasma is known. Dark circles highlight the protein ID's identified combining the proteomics data obtained using the fibers ENF001, ENF002, and ENF003 in parallel.
- Figure 10d.: Swarmplot that summarizes figures 10a-10c.
- Figure 11.: Plasma biomarkers identified by use of different types of fibers.
- Figure 12.: Unique proteins identified by combining the proteomic data obtained from different types of fibers and microparticles.
- Figure 13a.: Venn diagram shows unique protein identifications obtained by use of ENC003 with and without depletion preincubation.
- Figure 13b.: Venn diagram shows unique protein identifications obtained by use of ENC006 with and without depletion preincubation.
- Figure 14.: Venn diagram shows unique protein identifications obtained by use of ENC001, ENF002, and ENC017.

The Examples illustrates the invention.

### Example 1: Materials and methods

20 ul of human plasma (Diaserve Laboratories GmbH) was used for each experiment. Silica microparticles and fibers with varied surface chemical groups were obtained from Dr. Maisch HPLC GmbH (German patent application 10 2017 009 236.2). Except otherwise mentioned, consumables from the ENRICH-iST kit (PreOmics GmbH, P.O.00163) were used for protein incubation, binding, digestion, and clean-up.

### Preparation of microparticles.

Slurries of silica microparticles with different physical properties and surface chemistries (Table 1-2) were prepared separately by adding H2O to a final concentration of 10 mg/ml. 30 µL of each bead slurry was taken to a new reaction vessel after vortexing to homogeneous suspension. For reversed-phase microparticles (ENC011 - ENC016), the stock solution was prepared in 100% acetonitrile (MeCN) and 20% MeCN was added into the buffers used for bead washing and plasma incubation from the PreOmics kit. Other steps remained the same.

Microparticles were homogeneously dispersed in 200 µL of bead washing buffer from the PreOmics kit. The supernatant was then removed and discarded after 3-min centrifugation at 100 × g.

### Incubation of microparticles with the sample.

80-µL of binding buffer from the PreOmics kit and 20 µL of plasma were sequentially added to each pellet followed by proper mixing via pipetting. Each sample was then incubated in a thermoshaker at 30 °C and 1200 rpm for 30 min.

To remove unbound proteins, samples were centrifuged for 3 min at 100 × g, supernatant was removed, and 100 µL of binding buffer from the PreOmics kit was added. Subsequently, samples were brought into homogeneity again by end-to-end mixing, incubated in a thermoshaker at room temperature and 1200 rpm for 1 min, and centrifuged for 3 min at 100 × g. After removing the supernatant, the abovementioned wash steps were repeated twice for a total of three wash steps.

### Incubation of fibers with the sample

For testing fibers, the initial step of solubilization performed for microparticles was not necessary, which is an advantage in comparison with particles. 3 mg of each type of fiber (ENF001, ENF002 and ENF003) was directly and separately mixed with the plasma (parallel incubation). Also advantageous is that fibers were easily separated from the supernatant excluding any step of centrifugation, precipitation (gravity or magnetically assisted), instead the supernatant was removed simply by pipetting it off directly after incubation.

### Lysis and digestion.

For each resulting protein-microparticles pellet, 50 µL lyse buffer from the PreOmics kit was added and incubated in a thermoshaker at 95 °C and 1200 rpm for 10 min after proper mixing. After cooling down to room temperature, samples were subjected to digestion and clean-up according to the protocol of ENRICH-iST kit (PreOmics GmbH, P.O.00163).

### Data acquisition.

Purified and dried peptides were reconstituted in the LC-MS-suitable buffer from the PreOmics kit and peptide concentrations were determined using NanoDrop (A260). 300 ng of peptides were injected onto LC-MS system (EASY-nLC 1200 coupled to timsTOF Pro) in diaPASEF mode. The resulting data were processed using DIA-NN 1.8.

### One-pot workflow

A set of microparticles was prepared as it was previously described. The set of microparticles (ENC002, ENC003 and ENC006 or ENC001, ENC002, ENC003, ENC004 and ENC006) was mixed and incubated with plasma under the same conditions previously described.

### Sequential incubation workflow

ENC002, ENC003, and ENC006 microparticles were prepared as previously described. ENC002 microparticles were incubated with plasma under the same conditions previously described. The extracted supernatant was incubated again with microparticles ENC003. This supernatant was extracted and incubated again, with microparticles ENC006. All samples were further processed separately in the same manner previously described. The proteomic data of these samples was then combined.

### Sequential, crossed workflow

ENC008 and ENC006 microparticles are prepared as previously described. The 20 µL of plasma is divided into equal volumes, Aliquot 1 and Aliquot 2. In incubation A, ENC008 is incubated with Aliquot 1, and incubation B, ENC006 is incubated with Aliquot 2. Incubation A forms Supernatant A and Pellet A, the pellet containing a set of proteins bind to the microparticles. Incubation B forms Supernatant B and Pellet B. Then in incubation C, Supernatant A is incubated with Pellet B, and in incubation D, Supernatant B is incubated with Pellet A. The new pellets from incubation C and D are processed as previously described (lysis and digestion). The proteomic data obtained is then combined.

### Parallel incubation workflow

Microparticles were prepared as previously described. Fibers or microparticles were incubated under the same conditions previously described. Multiple types of fibers or microparticles were separately incubated with aliquots of the same sample. Lysis, digestion and data acquisition was performed as previously described. The identified unique proteins of example 2 corresponding to the set of microparticles including ENC002, ENC003 and ENC006 were combined, and the set of microparticles including ENC001, ENC002, ENC003, ENC004 and ENC006 were also combined. The identified unique proteins of fibers ENF001+ENF002 and ENF003 from Example 9 were combined.

### Depletion pre-incubation

ENC006, and ENC003 microparticles were prepared as previously described (Example 1). Mixtures of particles were formed, Mix 1 was ENC008 and ENC006, Mix 2 was ENC003 and ENC008. Every mixture is incubated with aliquots of 20 µL of the same plasma. The supernatants of every incubation are separately collected. Then, the supernatant of Mix 1 is incubated ENC003, and the supernatant from Mix 2 is incubated with ENC006. The new pellets are separately processed as previously described (Example 1). The proteomic data is obtained. The data can be combined with the data obtained by use of the same microparticles without depletion ("Incubation of fibers with the sample", example 2).

### Preferred materials (as used in the Examples below)

**Table 1. Silica microparticles with different functional groups at the surface**

| Code | Functional group |
|---|---|
| ENC001 | Silica silanol functionalized |
| ENC002 | Diol |
| ENC003 | CN |
| ENC004 | NH2 |
| ENC005 | 4-Ethylpyridin |
| ENC006 | Propyl-strong anion exchange |
| ENC007 | Si-C3H6-NH-CO-CH2-CH2-COOH |
| ENC008 | Si-C3H6-O-CH2-COH2-CH2-SO3H |
| ENC009 | Propyl-strong cation exchange |
| ENC010 | Carboxymethyl |
| ENC011 | Methyl |
| ENC012 | Ethyl |
| ENC013 | Butyl |
| ENC014 | Butyl+polar groups |
| ENC015 | Pentyl |
| ENC016 | Phenyl |
| ENC017 | Paramagnetic silica silanol functionalized |

**Table 2. Silica microparticles with different particle and pore sizes**

| Code | Particle size ( 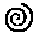 m) | Pore size (Å) |
|---|---|---|
| ENP001 | 3 | 120 |
| ENP002 | 10 | 120 |
| ENP003 | 10 | 300 |
| ENP004 | 10 | 1000 |

**Table 3. Fibers from different materials**

| Code | Fiber material | Chemical category |
|---|---|---|
| ENF001 | Quartz | SiO2 and derivatives |
| ENF002 | Silica silanol functionalized | SiO2 and derivatives |
| ENF003 | Cotton | Cellulose and derivatives |
| ENF004 | Viscose | Cellulose and derivatives |
| ENF005 | Sheep | Protein and derivatives |
| ENF006 | Silk | Protein and derivatives |
| ENF007 | Nylon | Polymer |
| ENF008 | Polyester | Polymer |
| ENF009 | Acrylic | Polymer |
| ENF010 | Polyethylene | Polymer |
| ENF011 | Polypropylene | Polymer |
| ENF012 | Aramid | Polymer |
| ENF013 | Polytetrafluoroethylene | Polymer |
| ENF014 | Ceramic | Metal and metal oxides |
| ENF015 | Aluminium | Metal and metal oxides |
| ENF016 | Carbon | Carbon |

**Table 4. Monoliths from different materials**

| Code | Monoliths | Chemical category |
|---|---|---|
| ENM001 | Si | Inorganic oxide |
| ENM002 | Zr | Inorganic oxide |
| ENM003 | Ti | Inorganic oxide |
| ENM004 | Al | Inorganic oxide |
| ENM005 | Hf | Inorganic oxide |
| ENM006 | C | Inorganic oxide |
| ENM007 | Au | Inorganic oxide |
| ENM008 | Ag | Inorganic oxide |
| ENM009 | Ce | Inorganic oxide |
| ENM010 | Ge | Inorganic oxide |
| ENM011 | Polymethacrylate | Polymer |
| ENM012 | Polyacrylamide | Polymer |
| ENM013 | Polystyrene | Polymer |
| ENM014 | Agarose | Polymer |
| ENM015 | Cellulose | Polymer |

### Example 2: functionalized microparticles increased the number of proteins identified.

Microparticles described in Table 1 were tested as described in Example 1.

Fig. 1 shows the unique proteins from human plasma that were identified using different silica microparticles. The number of identified proteins using the microparticle workflow increased by at least 119% and up to 290% when compared to plasma that was not incubated with a particle (neat plasma). Furthermore, particles that are functionalized led to a larger increase compared to silica particles (ENC001), up to 78% for ENC008. It is important to realize that there was no predictable correlation between functional group and number of identified proteins, as for example particle ENC008 led to an increase of 39% compared to particle ENC009, which both contain a sulfonic acid moiety.

### Example 3: The combination of microparticles in one pot increased the number of proteins identified.

The combination of microparticles with different functionalization in one-pot workflow with combined incubation led to an increase in unique protein identifications compared to the samples incubated with a single particle only (Fig. 2). The incubation of microparticles in one-pot has a further advantage, the reduction of amount of plasma and time required to operate the instruments and evaluate the data. In the case of 5 different particles, this resulted in a reduction of resources (amount of time and samples) of 400%.

### Example 4: The sequential incubation with multiple microparticles of a single sample increased the number of proteins identified.

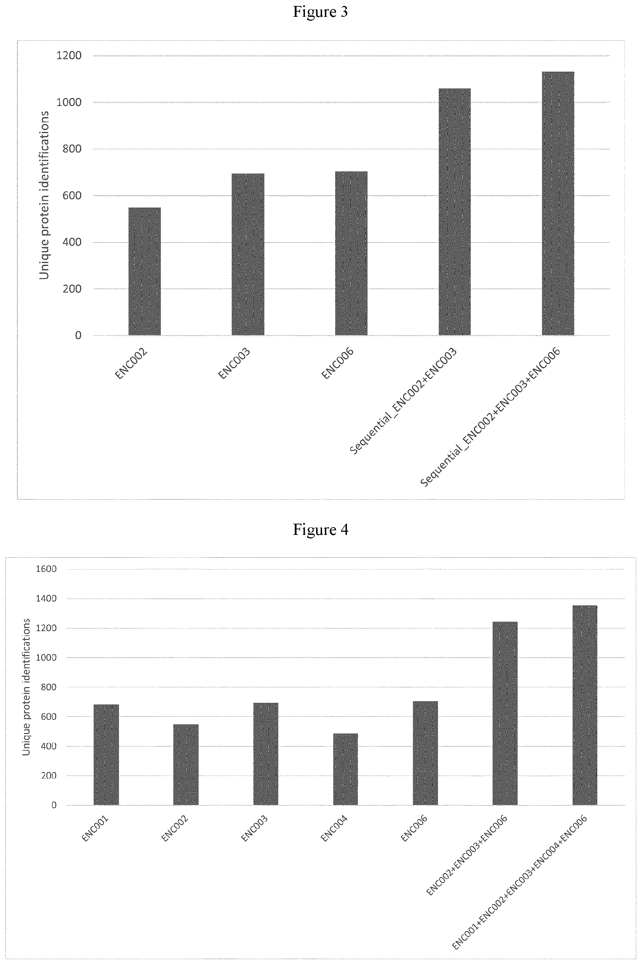

Compared to samples generated with single particles, the sequential assay increased the number of identified unique proteins (Fig. 3). This increase was already pronounced for only two different sequentially employed particles and increased further when three different particles where used. As an additional benefit, this approach reduced the amount of plasma needed compared to parallel sampling of the different particles.

### Example 5: The use of multiple microparticles in parallel incubation workflow (by combination of proteomic data) increased the number of proteins identified.

As shown in Fig. 4, the combination of unique protein identifications obtained from differently functionalized microparticles led to an increase of the overall number of proteins, demonstrating the orthogonality of the proteins bound to different particles.

### Example 6: Comparison of unique protein identifications obtained by the combination of proteomics data of multiple microparticles assayed in parallel and sequentially.

The identified unique proteins obtained in Example 4 and Example 5 were compared.

As can be seen in Fig. 5, the Venn diagram shows that the overlap of unique protein identifications between the combination of data generated separately using three different microparticles incubated in parallel and the same three microparticles incubated sequentially was only 60%. This highlighted the orthogonality of the two approaches and the benefit of performing both workflows to increase the information gained.

### Example 7: Smaller pore size and combination of proteomic data from microparticles with different pore sizes increased the number of proteins identified (parallel incubation).

Microparticles ENP002, ENP003 and ENP004 were tested as described in Example 1. In this case, a different plasma was used which makes the results obtained here incommensurable with the other examples.

Using silica microparticles led to an increase in unique protein identifications irrespective of the particle pore size as depicted in Fig. 6a when compared to neat plasma. The highest gain was achieved when using particles with a pore size of 120 Å, which showed an increase of 170%. Furthermore, the unique protein identifications from samples using particles with different pore sizes were combined (Fig. 6b). This showed the orthogonality of particles with different pore sizes, as the increase over neat plasma rose to 222% when combining all three particles.

### Example 8: The combination of microparticles with different particle sizes in one pot can increase the number of proteins identified.

At least two types of silica microparticles with differing particle sizes are combined in a reaction tube. These particles either have the same or differing functionalized surfaces. After binding of proteins from a biofluid as described in Example 1, the microparticles are separated by e.g. filtration through a filter with a pore size that allows for selective separation of the microparticle with the smallest size. This process can then be repeated using other filters with suitable pore sizes until all different microparticles are separated. Subsequently, the workflow is continued separately for each microparticle fractions with the steps as described in Example 1.

The resulting number of proteins identified when combining the data from all used particle sizes increases when compared to data from only a single particle type. When using different chemistries, the data is expected to be comparable to or better than Example 3, where it was shown that a one-pot reaction of multiple particles increases the number of identified proteins compared to silica microparticles ENC001 (Fig. 2) with the advantage being that the microparticles can be separately analyzed by mass spectrometry after separation. Furthermore, the data is expected to be comparable to or better than Example 5, where it was shown that a combination of the proteomic data from separately processed differently functionalized microparticles increases the number of identified proteins compared to silica microparticles ENC001 (Fig. 4) with the advantage being that a smaller amount of sample is required.

### Example 9: Fibers increased the number of proteins identified.

Fig. 7 shows that a larger number of unique protein identifications is achieved by incubation of aliquots of the same plasma with fibers, in comparison to neat plasma. The increase in unique protein identifications is of up to 172% (ENF002). This highlighted the benefit of using fibers to reach a better coverage of the proteome of bio-fluids.

### Example 10: Comparison of unique identified proteins between silica microparticles and silica-based fibers.

The overlap of unique proteins identified using silica-based microparticles ENC001 and any of the silica-based fiber, ENF001 (Fig. 8a) or ENF002 (Fig. 8b) was compared.

Surprisingly, the overlap of common proteins between data from the silica microparticle and both silica fibers, silica silanol functionalized and quartz (Fig. 8a and 8b) is around 41% in both cases, highlighting the non-obvious orthogonality of these solid phases from the same material.

Figure 8b compares the sets of unique proteins identified using silica silanol functionalized particles and silica silanol functionalized fibers, meaning that the chemical nature is the same and only the shape is different. Accordingly, Figure 8b make a proper comparison of the shapes keeping the chemical nature constant. Advantageously, Figure 8b demonstrates that compared with particles (642 unique proteins) the use of fibers (845 unique proteins) allows the identification of a higher total number of unique proteins, this means 32% more unique proteins than by the use of particles.

The same was observed in a further experiment wherein ENC001, ENF002 and a paramagnetic version of ENC001, ENC017 where compared (Fig. 14).

### Example 11: The combination of proteomic data of fibers incubated in parallel increased the number of proteins identified.

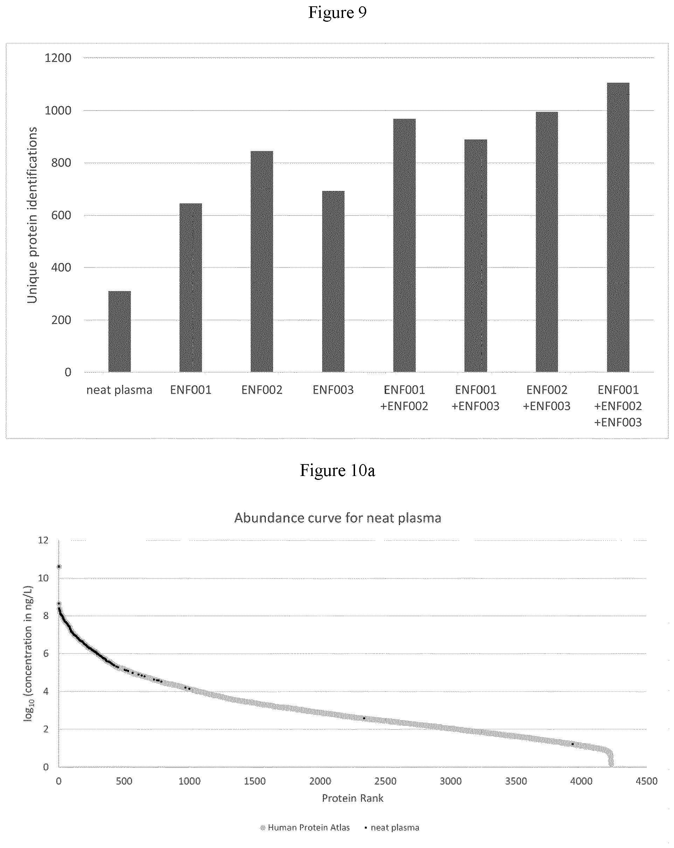

As shown in Fig. 9, combining the identified unique proteins obtained using different types of fibers resulted in an increase of overall unique proteins compared to the values for these solid phases on their own. This was the case for both combinations of two and three different types of fibers.

### Example 12: Fibers increased the coverage of low abundant plasma proteins.

The identified unique proteins of neat plasma, plasma incubated with ENF002 as well as a combination of the unique protein identifications from ENF001, ENF002 and ENF003 were compared to plasma's protein abundance data from the Human Proteome Atlas.

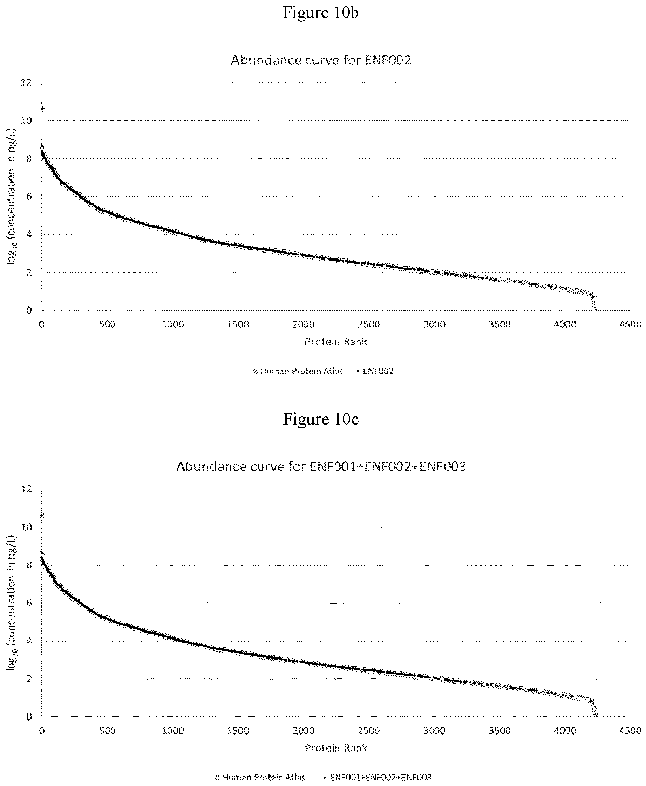

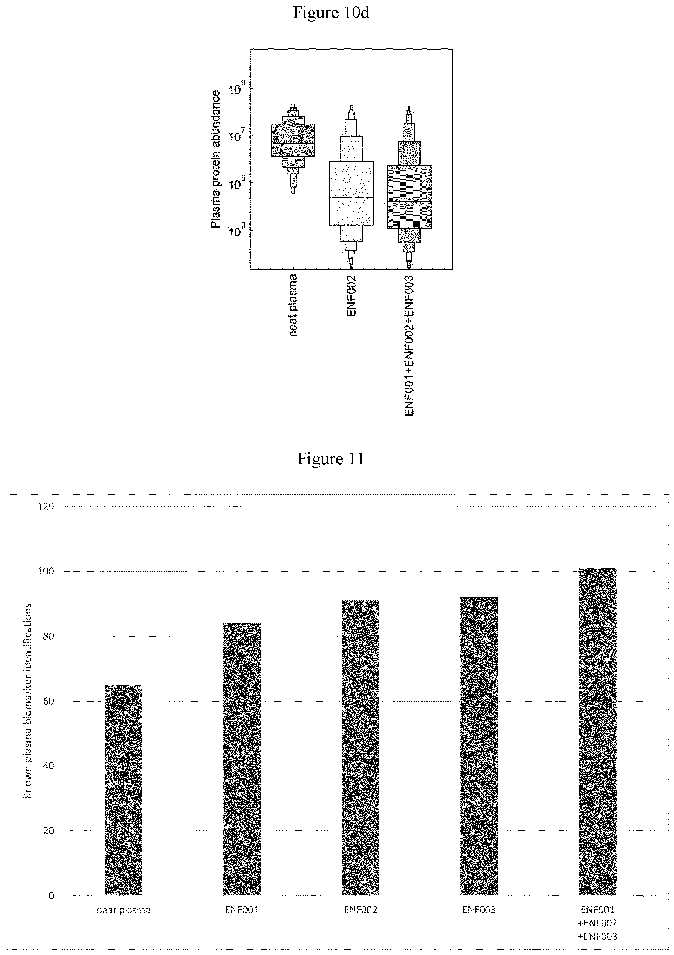

Fig. 10 a-c show abundance curves, and 10 d swarmplot for plasma proteins identified in neat plasma (Fig. 10a), plasma processed using ENF002 (Fig. 10b), and a combination of the proteomic data obtained from using ENF001, ENF002 and ENF003 in parallel. The unique proteins identified in the neat plasma analysis were almost exclusively high abundant proteins with a concentration of 105 ng/L or higher (Fig. 10a). In contrast to this, incubation of plasma with silica silanol functionalized fibers (ENF002) resulted in identification of many low abundant proteins down to 101 ng/L (Fig. 10b), which constitutes an improvement in sensitivity of 4 orders of magnitude when compared to neat plasma. The combination of proteomic data from three different types of fibers further increased the coverage of the low abundant proteome (Fig. 10c).

### Example 13: Fibers increased the number of identified known plasma biomarkers.

The proteomic data of neat plasma, plasma processed using either ENF001, ENF002 or ENF003 as well as a combination of the unique protein identifications from ENF001, ENF002 and ENF003 was searched for the presence of known biomarkers using a list of MS-identifiable proteins that are FDA-approved biomarkers.

The data depicted in Fig. 11 and Table 5 shows that fibers as ENF003 increased the number of biomarkers identified in plasma by 42% compared to neat plasma. Combining the data from three different fibers expanded the overall number of identified plasma biomarkers further resulting in an increase of 55% compared to neat plasma.

**Table 5: Uniprot Accession Numbers of FDA-approved biomarkers identified using different fibers.**

| **neat plasma** | **ENF001** | **ENF002** | **ENF003** | **ENF001 +ENF002 +ENF003** |
|---|---|---|---|---|
| P00450 | P00338 | O75874 | O75874 | O75874 |
| P00488 | P00450 | P00338 | P00338 | P00338 |
| P00734 | P00488 | P00450 | P00450 | P00450 |
| P00738 | P00505 | P00451 | P00451 | P00451 |
| P00740 | P00734 | P00488 | P00488 | P00488 |
| P00742 | P00738 | P00734 | P00734 | P00505 |
| P00747 | P00740 | P00738 | P00738 | P00734 |
| P00748 | P00742 | P00740 | P00740 | P00738 |
| P01008 | P00747 | P00742 | P00742 | P00740 |
| P01009 | P00748 | P00747 | P00747 | P00742 |
| P01019 | P00750 | P00748 | P00748 | P00747 |
| P01023 | P01008 | P00750 | P00750 | P00748 |
| P01024 | P01009 | P01008 | P01008 | P00750 |
| P01031 | P01019 | P01009 | P01009 | P01008 |
| P01034 | P01023 | P01019 | P01019 | P01009 |
| P02647 | P01024 | P01023 | P01023 | P01019 |
| P02649 | P01031 | P01024 | P01024 | P01023 |
| P02671 | P01034 | P01031 | P01031 | P01024 |
| P02675 | P01344 | P01034 | P01034 | P01031 |
| P02741 | P01889 | P01344 | P01236 | P01034 |
| P02745 | P02647 | P01889 | P01344 | P01236 |
| P02746 | P02649 | P02647 | P01889 | P01344 |
| P02747 | P02671 | P02649 | P02144 | P01889 |
| P02749 | P02675 | P02671 | P02647 | P02144 |
| P02751 | P02745 | P02675 | P02649 | P02647 |
| P02753 | P02746 | P02741 | P02671 | P02649 |
| P02760 | P02747 | P02745 | P02675 | P02671 |
| P02763 | P02749 | P02746 | P02741 | P02675 |
| P02765 | P02751 | P02747 | P02745 | P02741 |
| P02766 | P02753 | P02749 | P02746 | P02745 |
| P02768 | P02760 | P02751 | P02747 | P02746 |
| P02775 | P02763 | P02753 | P02749 | P02747 |
| P02776 | P02765 | P02760 | P02751 | P02749 |
| P02786 | P02766 | P02763 | P02753 | P02751 |
| P02787 | P02768 | P02765 | P02760 | P02753 |
| P02788 | P02775 | P02766 | P02763 | P02760 |
| P02790 | P02776 | P02768 | P02765 | P02763 |
| P03951 | P02786 | P02775 | P02766 | P02765 |
| P03952 | P02787 | P02776 | P02768 | P02766 |
| P04040 | P02788 | P02786 | P02775 | P02768 |
| P04070 | P02790 | P02787 | P02776 | P02775 |
| P04114 | P03951 | P02788 | P02786 | P02776 |
| P04275 | P03952 | P02790 | P02787 | P02786 |
| P04278 | P04040 | P03951 | P02788 | P02787 |
| P04439 | P04075 | P03952 | P02790 | P02788 |
| P05062 | P04114 | P04040 | P02818 | P02790 |
| P05109 | P04275 | P04070 | P03951 | P02818 |
| P05155 | P04278 | P04075 | P03952 | P03951 |
| P05160 | P04439 | P04114 | P04070 | P03952 |
| P06276 | P05019 | P04275 | P04075 | P04040 |
| P06702 | P05106 | P04278 | P04114 | P04070 |
| P07195 | P05109 | P04439 | P04275 | P04075 |
| P07225 | P05155 | P05019 | P04278 | P04114 |
| P07359 | P05160 | P05106 | P04439 | P04275 |
| P08519 | P05164 | P05109 | P05019 | P04278 |
| P08697 | P05556 | P05121 | P05106 | P04439 |
| P08709 | P06702 | P05155 | P05109 | P05019 |
| P0C0L4 | P06732 | P05160 | P05121 | P05106 |
| P0C0L5 | P07195 | P05164 | P05155 | P05109 |
| P12259 | P07225 | P05455 | P05160 | P05121 |
| P17936 | P07359 | P05556 | P05164 | P05155 |
| P19652 | P08514 | P06702 | P05556 | P05160 |
| P35030 | P08519 | P07195 | P06702 | P05164 |
| P43251 | P08697 | P07225 | P07195 | P05455 |
| P61626 | P08709 | P07359 | P07225 | P05556 |
| P61769 | P0C0L4 | P08514 | P07359 | P06702 |
| P68871 | P0C0L5 | P08519 | P08514 | P06732 |
| P69905 | P12259 | P08575 | P08519 | P07195 |
| | P12277 | P08697 | P08697 | P07225 |
| | P12532 | P08709 | P08709 | P07359 |
| | P13224 | P08727 | P0C0L4 | P08514 |
| | P14618 | P0C0L4 | P0C0L5 | P08519 |
| | P14770 | P0C0L5 | P12259 | P08575 |
| | P14780 | P11413 | P12532 | P08697 |
| | P16671 | P12259 | P13224 | P08709 |
| | P17936 | P13224 | P14618 | P08727 |
| | P19652 | P14618 | P14770 | P0C0L4 |
| | P35030 | P14770 | P14780 | P0C0L5 |
| | P48735 | P14780 | P16671 | P11413 |
| | P61626 | P16671 | P17936 | P12259 |
| | P61769 | P17301 | P19652 | P12277 |
| | P68871 | P17936 | P24158 | P12532 |
| | P69905 | P19652 | P35030 | P13224 |
| | Q13093 | P35030 | P40197 | P14618 |
| | | P40197 | P43251 | P14770 |
| | | P48735 | P48735 | P14780 |
| | | P61626 | P61626 | P16671 |
| | | P61769 | P61769 | P17301 |
| | | P68871 | P68871 | P17936 |
| | | P69905 | P69905 | P19652 |
| | | Q13093 | Q13093 | P24158 |
| | | | Q9NZQ7 | P35030 |
| | | | | P40197 |
| | | | | P43251 |
| | | | | P48735 |
| | | | | P61626 |
| | | | | P61769 |
| | | | | P68871 |
| | | | | P69905 |
| | | | | Q13093 |
| | | | | Q9NZQ7 |

### Example 14: The combination of proteomic data of fibers and microparticles increased the number of proteins identified.

The identified unique proteins of ENC008 (Example 2) and fibers ENF001, ENF002 and ENF003 (Example 9) were combined.

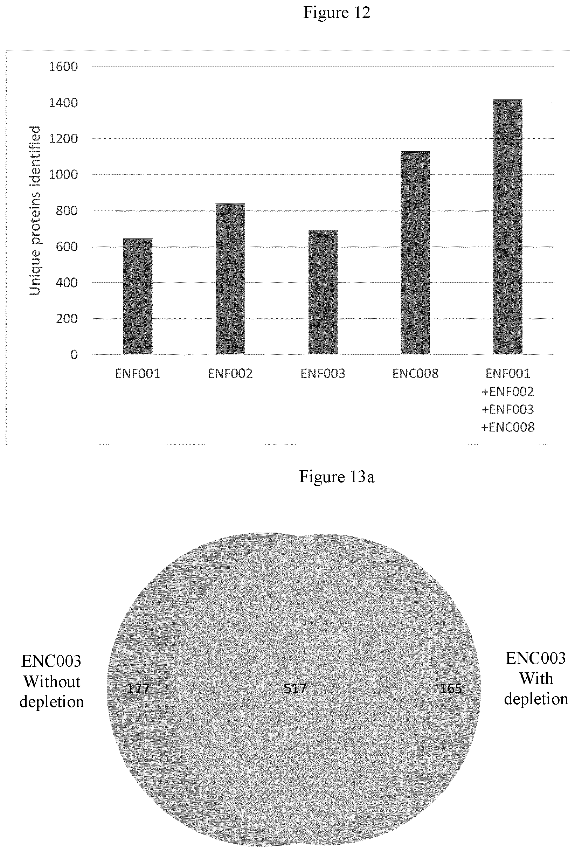

The combination of unique protein identifications from a particle and different fibers increased the total number of covered plasma proteins (Fig. 12). This demonstrates that the orthogonality, also seen in Example 10 contributes to the identification of a higher number of unique protein ID's.

### Example 15: The combination of fibers and microparticles in one pot increases the number of proteins identified.

At least two different solid phases from Table 1, Table 2 and Table 3, of which at least one is a fiber, are combined into a single reaction vessel and incubated with a biofluid as described in Example 1. If desired, the solid phases can be separated after incubation by either transferring the supernatant into a new reaction vessel (when fibers and microparticles are combined), by separating the solid phases mechanically, e.g., by using tweezers (when multiple fibers are combined) or by a combination of those techniques (when multiple fibers and microparticles are combined). The samples (with or without separation of the solid phases) are then further processed as described in Example 1. In case of separation of the solid phases, the proteomic data of all samples is combined.

The number of proteins identified with this workflow increases compared to using only a single fiber or microparticle. This is expected, as it was shown that the combination of differently functionalized silica microparticles in one pot increased the number of identified proteins compared to a single microparticle (Fig. 2). The approach described here has the advantages that it expands the range of usable solid phases to fibers and that the solid phases can be separately analyzed by mass spectrometry.

### Example 16: The sequential assaying of a single sample with fibers and microparticles increases the number of proteins identified.

At least two different solid phases from Table 1, Table 2 and Table 3, of which at least one is a fiber, are sequentially incubated with a biofluid as described in Example 4 and then further processed separately as described in Example 1. The proteomic data of these samples is combined.

The resulting number of proteins is expected to be comparable to or higher than when the proteomic data from different fibers (Fig. 8) or fibers and microparticles (Fig. 9) that were analyzed completely separately is combined. This is based on the fact that the sequential approach using differently functionalized silica microparticles (Fig. 3) gives comparable results to the combination of proteomic data of the same microparticles being analyzed completely separately.

### Example 17: Silica silanol functionalized fiber filters increase the number of proteins identified.

A biofluid sample is incubated in a reaction vessel that contains a silica silanol functionalized fiber filter membrane at the bottom. The supernatant is pushed through the filter, leaving proteins bound to the filter membrane behind. The filter membrane is then washed to remove unspecifically bound and/or residual unbound proteins. The proteins bound to the filter are then digested to peptides. These peptides are analyzed by mass spectrometry.

The resulting number of identified proteins increases compared to a mass spectrometric analysis of neat plasma. The data is expected to be comparable to the results for fiber ENF002 shown in Fig. 7a with the advantage that it allows for easy automation and parallelization of the workflow when using a filter plate with multiple reaction vessels.

### Example 18: Depletion pre-incubation increases the number of proteins identified.

As shown in Fig. 13a, depletion with the mixture ENC008 and ENC006, and processing of the supernatant with ENC003 allows the identification of 165 unique proteins that were not detected in the experiment without depletion (example 2), this represents a 24% of the set of unique proteins obtained without depletion for ENC003.

As shown in Fig. 13b, depletion with the mixture ENC008 and ENC003, and processing of the supernatant with ENC006 allows the identification of 146 unique proteins that were not detected in the experiment without depletion (example 2), this represents a 20% of the set of unique proteins obtained without depletion for ENC006.

This example demonstrates that is possible to enlarge the number of unique proteins detected with a matrix by previously depleting the sample. The combination of both methods, with and without depletion allowed an increase of 20-24% in the number of unique proteins detected.

## Claims

1. A method of reducing the dynamic range of biomolecules in a biological fluid, said method comprising:
bringing said biological fluid into contact with fibers;
wherein biomolecules as bound to said fibers are **characterized in that** said dynamic range is reduced as compared to said biological fluid.

2. The method of claim 1, wherein
(a) said biomolecules are proteins, polypeptides and/or peptides;
(b) said biological fluid is selected from bodily fluid, cell lysate, cell homogenate and processed forms of any of these; preferably bodily fluid, said bodily fluid preferably being selected from plasma, serum, full blood, cerebrospinal fluid, saliva, nasal swab, urine, synovial fluid, vaginal fluid and semen; and/or
(c) said fibers are selected from mineral fibers such as silica fibers, glass fibers and quartz fibers; organic or textile fibers such as cellulose, cotton, wool, hemp, linen, proteins and polymers; and metal or metal oxide fibers; preferably silica fibers, glass fibers and quartz fibers.

3. The method of claim 1 or 2, wherein said method comprises analysis and/or quantitation of said biomolecules as bound to said fibers, wherein preferably said analysis and/or quantitation is done by mass spectrometry.

4. The method of any one of the preceding claims, wherein said biomolecules are
(a) proteolytically digested while being bound to said fibers; or
(b) eluted from said fibers after said bringing into contact.

5. The method of any one of the preceding claims, wherein
(a) more than one type of fibers is used; or
(b) in addition to at least one type of fibers, microparticles, nanoparticles and/or monoliths are used, wherein preferably said microparticles or nanoparticles are mixed-phase particles and/or are porous with a preferred pore width of about 10 to 20 nm.

6. The method of any one of the preceding claims, wherein said method is repeated with the same or a different type of fiber, wherein, instead of said biological fluid, an eluate from the fibers or a supernatant as obtained in a preceding performance of said method is used.

7. The method of any one of the preceding claims, wherein a supernatant is removed, preferably by pipetting, aspirating or decanting only and/or removing said supernatant does not involve centrifugation.

8. The method of any one of the preceding claims, wherein said method comprises analyzing the data obtained from said analysis and/or quantitation, wherein preferably said analyzing comprises combining and/or comparing the said data obtained from a plurality of performances of the method of any one of claims 1 to 7.

9. The method of any one of the preceding claims, wherein furthermore the amount of substances interfering with analysis is reduced upon binding, said interfering substances including salts, sugars, amino acids, and, to the extent said biomolecules are proteins, polypeptides and/or peptides, said interfering substances include furthermore nucleic acids and lipids.

10. Use of fibers for reducing the dynamic range of biomolecules in a biological fluid.

11. A method of reducing the dynamic range of proteins, polypeptides and/or peptides in a biological fluid, said method comprising:
bringing said biological fluid into contact with microparticles or nanoparticles in a buffered aqueous solution with a pH value between about 7 and about 10;
wherein the particles are mixed-phase particles; and
wherein proteins, polypeptides and/or peptides as bound to the particles are **characterized in that** they are in their native state and that said dynamic range is reduced.

12. The method of claim 11, wherein said mixed-phase particles have normal phase and ion exchange properties, normal phase preferably being silica and ion exchange being preferably selected from WCX, SCX, WAX and SAX, wherein particularly preferred are silica particles with Si-C3H6-NH-CO-CH2-CH2-COOH, Si-C3H6-O-CH2-COH2-CH2-SO3H, Si-C3H6-O-CH2-COH2-CH2-NR1R2, or Si-C3H6-O-CH2-COH2-CH2-NR1R2R3 groups on their surface, wherein R1, R2 and R3 are independently H or C1 to C4 alkyl.

13. A method of reducing the dynamic range of proteins, polypeptides and/or peptides in a biological fluid, said method comprising:
bringing said biological fluid into contact with monoliths in a buffered solution with a pH value between about 7 and about 10;
wherein biomolecules as bound to said monoliths are **characterized in that** they are in their native sate and that said dynamic range is reduced.

14. The method of claim 13, wherein said monoliths comprise or consist of an oxide of an element selected from Si, Zr, Ti, Al, Hf, C, Au, Ag, Ce, and Ge; or of a polymer selected from polymethacrylate, polyacrylamide, polystyrene, agarose and cellulose, and preferably comprise or consist of silica.

15. A method of reducing the dynamic range of proteins, polypeptides and/or peptides in a sample and/or increasing the number of detectable proteins, polypeptides and/or peptides in a sample, said method comprising:
(a) adding an aliquot of said sample to each of two vessels, wherein each vessel contains a different binding matrix;
(b) allowing binding of proteins, polypeptides and/or peptides to each matrix;
(c) removing the supernatants from each vessel; and
(d) adding the supernatant removed from the first vessel of said two vessels to the second vessel and vice versa.

16. A method of reducing the dynamic range of proteins, polypeptides and/or peptides in a sample and/or increasing the number of uniquely detectable proteins, polypeptides and/or peptides in a sample, said method comprising:
(a) providing three different matrices in six different vessels as follows:
| | |
|---|---|
| vessel 1: | matrices 1 and 2 |
| vessel 2: | matrix 3 |
| vessel 3: | matrices 2 and 3 |
| vessel 4: | matrix 1 |
(b) adding an aliquot of said sample to each of vessels 1 and 3;
(c) allowing binding of proteins, polypeptides and/or peptides to the matrices in each of the vessels of (b); and
(d) transferring the supernatants from vessels 1 and 3 to vessels 2 and 4, respectively.

17. A kit comprising or consisting of:
(a) a protease;
(b) a reducing agent; and
(c) silica fibers, preferably glass wool or quartz wool, wherein said fibers comprise an outer layer comprising or consisting of material selected from mixed-phase material, said mixed phase material preferably being combined normal phase material; ion exchange material; reversed-phase material; normal phase material; a chelator; and any combination thereof.

18. A kit comprising or consisting of:
(a) a protease;
(b) a reducing agent; and
(c) a monolith comprising or consisting of silica, wherein said monolith comprises an outer layer comprising or consisting of material selected from mixed-phase material, said mixed phase material preferably being combined normal phase material; ion exchange material; reversed-phase material; normal phase material; a chelator; and any combination thereof.
